# EUROPEAN PATENT SPECIFICATION

(11) **EP 3 493 686 B1**
(45) Date of publication and mention of the grant of the patent: **27.03.2024**
(21) Application number: 17736937.8
(22) Date of filing: 06.07.2017
(51) Int. Cl.: A23L 33/00, A23L 33/21, A23L 33/135, A61K 31/702, A61K 31/733, A61K 35/745, A61P 1/12

(54) **NUTRITIONAL COMPOSITIONS AND INFANT FORMULAS COMPRISING A MIX OF OLIGOSACCHARIDES AND OPTIONALLY BIFIDOBACTERIUM LACTIS FOR PREVENTING, TREATING OR REDUCING THE SEVERITY OF NON-ROTAVIRUS-ASSOCIATED DIARRHOEA**
NÄHRSTOFFZUSAMMENSETZUNGEN UND SÄUGLINGSFORMELN MIT EINER MISCHUNG VON OLIGOSACCHARIDEN UND GEGEBENENFALLS BIFIDOBACTERIUM LACTIS ZUR VERHINDERUNG, BEHANDLUNG ODER REDUZIERUNG DER NICHT-ROTAVIRUS-ASSOZIIERTEN DIARRHÖE
COMPOSITIONS NUTRITIVES ET FORMULES POUR NOURRISSONS CONTENANT UN MÉLANGE D'OLIGOSACCHARIDES ET ÉVENTUELLEMENT BIFIDOBACTERIUM LACTIS POUR PRÉVENIR, TRAITER OU RÉDUIRE LA SÉVÉRITÉ DES DIARRHÉES NON ASSOCIÉES AU ROTAVIRUS

(30) Priority: 04.08.2016 EP 16182797
(43) Date of publication of application: 12.06.2019
(73) Proprietor: Société des Produits Nestlé S.A., 1800 Vevey (CH)
(72) Inventor: BERGER, Bernard, 1613 Maracon (CH); BRUESSOW, Harald, 1814 La Tour-de-Peilz (CH)
(74) Representative: Rupp, Christian
(86) International application number: PCT/EP2017/067019
(87) International publication number: WO 2018/024440

(56) References cited:
- WO-A1-2015/071402
- WO-A1-2017/021479
- US-A1- 2003 072 865
- US-A1- 2009 041 736
- US-A1- 2011 064 707
- US-A1- 2012 171 166
- US-A1- 2014 255 543

## Description

### Field of the Invention

The present invention relates to nutritional compositions for infants and young children and their health effects in infants. In particular, it relates to an infant formula comprising a specific mix of oligosaccharides probiotic for preventing, treating or reducing the severity or risk of diarrhoea and especially non-rotavirus-associated diarrhoea. In a further aspect the composition also comprises a probiotic Bifidobacterium animalis ssp lactis.

### Background to the Invention

Whenever mothers cannot breast-feed their infants, infant formula provides a suitable alternative to natural breast feeding with human breast milk. Nutritional compositions for infants and young children are often sold as powders to be reconstituted with water or in some instances as ready to drink or concentrated liquid compositions. Those compositions are intended to cover most or all the nutritional needs of the infants or young children.

It is known however, that human breast milk represents the ultimate gold standard in terms of infants' nutrition. Infant formula manufacturers have made many attempts to induce nutritional health effects close to or similar to the benefits of human breast milk.

Mother's milk is recommended for all infants. However, in some cases breast feeding is inadequate or unsuccessful for medical reasons or the mother chooses not to breast feed. Infant formulae have been developed for these situations. Fortifiers have also been developed to enrich mother's milk or infant formula with specific ingredients.

In many instances however, studies have shown that infant formula do not induce the identical effects on the body compared to human breast milk. For example, infants fed infant formula and infants fed human-breast milk (HBM) can exhibit a different intestinal (gut) microbiota.

Infancy, especially the first weeks, 3 months, 6 months or 12 months of life is critical for the establishment of a balanced gut microbiota.

It is know that the modulation of the gut microbiota during infancy can prospectively have a great influence in the future health status of the bodies. For example the gut flora can have influence on the development of obesity later in life, on the development of a strong immune system later in life or the normal growth.

Similarly, a healthy intestinal flora is an indicator of the health of an infant and altered intestinal microbiota can be an indicator (and/or a cause) of abnormal health events such as diarrhoea, under-absorption of nutrients, colic, altered sleep, and altered growth and development.

It is known that the mode of delivery can affect the initial gut microbiota of infants : infants delivered by Caesarean section (C-section) have been shown to have a different gut microbiota compared to vaginally-delivered infants.

It is known that, among other ingredients, non-digestible carbohydrates (prebiotics) in particular can affect the promotion of particular microbiota. For example, it has been shown that certain galacto-oligosaccharides (GOS) and/or certain fructo-oligosaccharides (FOS) can promote the growth and prevalence of bifidobacteria in the gut, especially in infants.

The gut microbiota and its evolution during the development of the infant is, however, a fine balance between the presence and prevalence (amount) of many populations of gut bacteria. Some gut bacteria are classified as "generally positive" while other are "generally negative" (or pathogenic) as to their effect on the overall health of the infant.

Certain species of "generally positive" bacteria, such as bifidobacteria, may be underrepresented in infants fed conventional infant formula in comparison to breast fed infants. Similarly some bacterial populations are considered pathogenic and should remain of low prevalence in the gut microbiota.

Indeed infant fed infant formulae may not benefit from the natural, well balanced intestinal gut flora (gut microbiota) of infants fed exclusively or predominantly Human Breast Milk. Such natural microbiota observed in breast fed infants is indeed both well controlled over time (evolution over time) and very complex. Many taxa of microorganisms co-exist in the highly complex microenvironment of the gut/intestine, each in sequentially defined proportions. Quantitative and qualitative dimensions are to be considered when defining the microbiota of infants or young children. Furthermore, the variation over time of the gut microbiota adds to the complexity.

A healthy intestinal flora is an indicator of the health of an infant and an altered intestinal microbiota can be an indicator (and/or a cause) of abnormal health events such as diarrhea, under-absorption of nutrients, colic, altered sleep and/or altered growth and development.

Diarrhea, also spelled diarrhea, is the condition of having at least three loose or liquid bowel movements each day. It often lasts for a few days and can result in dehydration due to fluid loss. Signs of dehydration often begin with loss of the normal stretchiness of the skin and changes in personality. This can progress to decreased urination, loss of skin color, a fast heart rate, and a decrease in responsiveness as it becomes more severe. Diarrhea also involves a dysbiosis of the global gut microbiota that precedes and follows the diarrhea event(s). Diarrhea is a major cause of childhood mortality, ranking among the top four largest contributors to years of life lost in sub-Saharan Africa and South Asia. The proportion of deaths attributed to diarrhea among children aged under 5 years is estimated to be approximately 15% worldwide. The most common cause is an infection of the intestines due to either a virus, bacteria, or parasite; a condition known as gastroenteritis. Rotavirus -induced or -related diarrhea is one of the major cause of diarrhea. *Escherichia coli* is after rotavirus a major pathogen of diarrhea in children from developing countries. Survival also carries risks. Frequent cases of diarrhea before age 2 years are linked with a subsequent average growth shortfall of 3.6 cm, elevated heart rate after exercise, a loss of 10 IQ points, and roughly a year delay in starting school. Thus, it is important to reduce the diarrhea episodes, but also to improve the recovery rate and the convalescence after them.

This type of enteric infections are generally self-limited conditions, but non-specific therapy can provide relief for some patients, and specific therapy may shorten the duration of the illness and eradicate fecal shedding of the organism. In caring for patients with diarrhea and dehydration, the major therapeutic considerations include fluid and electrolyte therapy, dietary manipulation (but restoration of feeding is important to reduce the nutritional defects), non-specific therapy with antidiarrheal compounds (to reduce symptoms) and specific therapy with antimicrobial agents (generally only in case of dysentery).

However these existing solutions are not very appropriate for infants and young children due to their young age and fragility, especially the use of antidiarrheal compounds is not approved for young children under 2 years old.

Antibiotics are also not appropriate because of the young age of infants and young children, but also because E.coli is resistant against many antibiotics (Jiang ZD, et al. Prevalence of enteric pathogens among international travelers with diarrhea acquired in Kenya (Mombasa), India (Goa), or Jamaica (Montego Bay). J Infect Dis. 2002;185(4):497-502).

Alternative therapies have therefore been developed.

Phage therapy was investigated against *E.coli* diarrhea (Brüssow H. http://www.ncbi.nlm.nih.gov/pubmed/16000704). Phage cocktails against many bacterial infections, including *E. coli* diarrhea, are sold in Russian pharmacies, but their efficacy has not been documented in detailed scientific reports. Neither T4-like nor a commercial Russian coliphage cocktail had any impact on quantitative diarrhea criteria in a pilot clinical trial with children mostly infected by enterotoxigenic *E.coli* (ETEC). Fecal ETEC peak was short-lived, low-tittered and only half of fecal *E. coli* isolates were phage-sensitive.

The use of probiotics has especially been investigated. For example *Lactobacillus paracasei* strain ST11 ameliorates the outcome of non-rotavirus diarrhea in children from Bangladesh but it has no effect on rotavirus diarrhea, A. Sarker, Pediatrics 2005. Probiotics do not consist of the most appropriate solution to treat or prevent diarrhea in a whole extend as they may only increase specific microbiota taxa. Probiotics are indeed considered to be viable microbial preparations which promote the individual's health by preserving the natural microflora in the intestine. They are deemed to attach to the intestine's mucosa, colonize the intestinal tract and likewise prevent attachment of harmful microorganisms thereon. A crucial prerequisite for their action resides in that they have to reach the gut's mucosa in a proper and viable form and do not get destroyed in the upper part of the gastrointestinal tract, especially by the influence of the low pH prevailing in the stomach. Another difficulty is that gut microbiota is very diversified and complex, and bacteria have various interactions in-between.

Other ingredients like non-digestible carbohydrates (prebiotics) have also been explored. Human Milk Oligosaccharides (HMOs) have especially been reported as reducing the duration of rotavirus diarrhea in formula-fed pigs (Li et al ISME J 2014 and a study from University of Illinois of 2015).

Siallyllactose has also been studied for an possible protective effect against diarrhoea. A. Weiss and T. Hennet have described "The role of milk sialyllactose in intestinal bacterial colonization" (Adv Nutr. 2012 May; 3(3): 483S-488S; Published online 2012 May 4. doi: 10.3945/an.111.001651).

WO200440032639A1 describes the combination of Fructo-oligosaccharides (FOS) / Oligofructose (FOS) and specific probiotics and siallyllactose which are useful in the eradication of pathogenic microorganisms in the gastrointestinal tracts of patients.

Bovine Colostrum has also been described as a natural treatment for Diarrhea and other Gastrointestinal Ailments.

*The review "*European Journal of Clinical Nutrition (2003) 57, 1351-1369. doi:10.1038/sj.ejcn.1601704", provides an overall view on the role and effect of sialyl acid in human nutrition.

WQ2012160080A1 by Berrocal et al, describes a mix of oligosaccharides and some of its effects without linking them to any particular beneficial effect such as diarrhea and especially non-rotavirus associated diarrhoea.

In the publication Nutrients 2011, 3, 228-244; doi:10.3390/nu3020228, D. Bergner et al., Sialyllactose was coupled to dipalmitoylphosphatidylethanolamine (PE) by reductive amination and the product (SLPE) purified by HPLC, and was tested against rotavirus associated diarrhea in piglets.

Certain of these substances may function as soluble decoy receptors in the gut, protecting the neonate from enteric pathogens (Newburg et al, Human milk glycans protect infants against enteric pathogens." Annual Review of Nutrition 2005;25:37-58) and they may also directly interact with gut epithelial cells yielding changes that may interfere with host-microbial interactions (Bode et al, 2012).

US 2009/0041736 A1 describes preparations comprising a probiotic and a prebiotic mixture which is specifically designed to enhance the efficiency and the efficacy of the probiotic.

US 2011/0064707 A1 is directed to the administration to infants without siblings of probiotic bacteria capable of promoting an early bifidogenic gut microflora.

WO 2017/021479 A1 is a document according to Art. 54(3) EPC and describes infant formula comprising a specific probiotic for inducing a gut microbiota that is close to the one of infants fed exclusively human breast milk.

While many attempts have been made, it remains a need to prevent and/or reduce the severity or duration of diarrhea, especially in infants in young children.

There is a specific need to identify compounds naturally present in conventional nutritional ingredients (such as bovine milk), and to associate them into a mix able to deliver particular health benefit against diarrhea and especially non-rotavirus associated diarrhea.

Most of these studies provide solutions that do not target the treatment or prevention of non-rotavirus diarrhea conditions and the entire associated symptoms. They may act on the microbiota only by modulating specific microbiota taxa, e.g. they result in an increased number of bifidobacteria or a decreased number of clostridia.

However, no solution is currently available to treat or prevent non-rotavirus diarrhea by bringing the global gut microbiota (i.e. the overall/entire/total/whole microbiota) closer to the normal. No existing solutions seem to also take into account the gut microbial function.

There is therefore a need for infants or young children to reduce the risks of diarrhea and /or non-rotavirus diarrhea and/or to improve the recovery by shortening the duration of the diarrhea (and non-rotavirus diarrhea) and/or by reducing the symptoms and consequences of diarrhea (and non-rotavirus diarrhea), especially by acting on the dysbiosis of the global gut microbiota preceding, during and/or following the diarrhea (or non-rotavirus diarrhea) events in said infants or young children.

There is a need to compensate for the abnormal overall gut microbiota observed in infants or young children in case of diarrhea or non-rotavirus diarrhea. There is a need to rebalance such overall gut microbiota, both in terms of composition and function.

There is a need to enhance a good balance in the overall gut microbiota of infants, especially by down-regulating or repressing the growth of pathogenic bacteria, during the first weeks of life when such a balance is being established.

There is a need for nutritional compositions for infants or young children that provide with a global gut microbiota and a metabolic signature closer to the ones obtained in normal situations (i.e. healthy, non-diarrhea situation) and/or for breast-fed infants.

There is a need to provide said infants or young children with the best nutrition that enables the development of a global gut microbiota close to the one not having non-rotavirus diarrhea and/or close to the one of breast-fed infants, said development being short term (i.e. during the nutritional intervention) and/or long term (i.e. after the nutritional intervention).

There is a need for nutritional compositions for infants or young children that provide a microbiota composition which is not permissive for the development of diarrhea or non-rotavirus diarrhea and/or which promotes a faster recovery from diarrhea / non-rotavirus diarrhea.

There is a need to deliver such health benefits in these infants or young children in a manner that does not induce side effects and/or in a manner that is easy to deliver, and well accepted by the parents or health care practitioners.

There is a need, for infants fed with infant formula, to promote and/or induce over time a microbiota that evolve in a similar manner as the microbiota of breast-fed infants, and thus positively influencing the events of diarrhea.

There is also a need, for infants fed with infant formula, to provide them with the best nutrition that enables the development of a microbiota close to the microbiota of breast-fed infants, short term during the nutritional intervention and/or long term, after the nutritional intervention.

There is a need to compensate for the sub-normal microbiota observed in non-breast-fed infants. There is a need to rebalance such microbiota.

There is a need, for infants fed with infant formula, to induce and/or promote the development of specific bacterial families, genus or species or strains in their intestine, such as to obtain a microbiota close to the microbiota of breast-fed infants which is naturally best to prevent or treat diarrhoea, especially non-rotavirus associated diarrhoea.

There is a need to down-regulate the development and growth of pathogenic bacteria or "negative bacteria" in the gut of infants and young children, while preferably also promoting healthy positive bacteria.

There is a need to selectively affect or reduce the growth of pathogenic bacteria in the gut of infants and young children while promoting or at least not impacting the growth of positive gut bacteria.

There is a need to enhance a good balance in the overall gut microbiota of infants, especially by down-regulating or repressing the growth of pathogenic bacteria, during the first weeks of life when such a balance is being established.

There is a need to enhance such good balance by well tolerated, "soft-impact" means having no or little side effects.

### Summary of the invention

The invention relates to a nutritional composition for infants and young children for its use, as defined by claim 1. The composition can also comprise a probiotic *Bifidobacterium animalis* spp. *lactis* probiotic. The infants or young children are between 0 and 36 months, preferably between 0 and 12 months of age, more preferably between 0 and 6 months of age. The composition of the invention is for use in treating or preventing or reducing the risk, and/or severity and/or occurrence of non-rotavirus-associated diarrhoea.

### Brief Description of the Figures

**Figures 1****:** shows proportion of stools of various consistency in different groups (group "T" : Test group receiving the composition of the invention, "B" Breast-fed group, control group "C" conventionally fed not according to the invention).
**Figure 2****:** shows the average weighted Unifrac distance between the formula-fed groups T and C and the breast-fed group B. Group T : with *B. lactis* probiotic; Group C: control without *B. lactis* probiotic. The nearer the distance to zero, the closer to the breast-fed group B.
**Figure 3****:** Boxplots of alpha-diversity analyses at three time points for the three feeding groups. P-value: *, <0.05; **, <0.01; ***, <0.001 (group T : with *B. lactis* probiotic; group C : control without *B. lactis* probiotic; group B : breast-fed)
**Figure 4****:** Bacterial equivalent counts measured by qPCR in the feces of infants receiving the specified formula (C=control; T= test formula with *B. lactis* probiotic) or who were breastfed (group B) at enrolment (<2w) and at six (6w) and twelve (12w) weeks of age. qPCR results are given as means + sem of bacterial cells per g stool. Dotted line, detection limit. error bars, standard deviation.
**Figure 5****:** Proportion of infants in the three feeding groups specified at the bottom showing stools with the colour defined on the abscissa at <2 w (baseline) and 12 w of age (right panel). (group T : with *B. lactis* probiotic; group C : control without *B. lactis* probiotic; group B : breast-fed)
**Figure 6****:** Bifidobacteria counts (log CFU of bifidobacteria per g of feces) in infants at 3, 10, 28 and 84 days, in each group tested (test formula/vaginal delivery; test formula/caesarean delivery; control formula/vaginal delivery; control formula/caesarean delivery).

### Description of the invention

### Definitions :

As used herein, the following terms have the following meanings.

The term "infant" means a child under the age of 12 months.

The expression "young child" means a child aged between one and three years, also called toddler.

An "infant or young child born by C-section" means an infant which was delivered by caesarean section. It means that the infant was not vaginally delivered.

A "preterm" or "premature" means an infant or young child that was not born at term. Generally it refers to an infant born prior to the completion of37 weeks of gestation.

The expression "nutritional composition" means a composition which nourishes a subject. This nutritional composition is usually to be taken enterally, orally, parenterally or intravenously, and it usually includes a lipid or fat source and a protein source. Preferably, a nutritional composition is for oral use.

The expression "hypoallergenic nutritional composition" means a nutritional composition which is unlikely to cause allergic reactions.

The expression "synthetic composition" means a mixture obtained by chemical and/or biological means, which can be chemically identical to the mixture naturally occurring in mammalian milks.

The expression "infant formula" means a foodstuff intended for particular nutritional use by infants during the first four to six months of life and satisfying by itself the nutritional requirements of this category of person (Article 1.2 of the European Commission Directive 91/321/EEC of May 14, 1991 on infant formulae and follow-on formulae).

The expression "starter infant formula" means a foodstuff intended for particular nutritional use by infants during the first four months of life.

The expression "follow-on formula" means a foodstuff intended for particular nutritional use by infants aged over four months and constituting the principal liquid element in the progressively diversified diet of this category of person.

The expression "baby food" means a foodstuff intended for particular nutritional use by infants during the first years of life.

The expression "fortifier" refers to liquid or solid nutritional compositions suitable for mixing with breast milk or infant formula.

The term "weaning period" means the period during which the mother's milk is substituted by other food in the diet of an infant.

The "mother's milk" should be understood as the breast milk or colostrum of the mother (= Human Breast Milk = HBM).

The term "oligofructose" as used herein refers to a fructose oligomers. It can be long chain or short chain , pending on the degree of polymerization of the oligofructose (number of monomers). Preferably the oligofructose of the invention is a short-chain oligofructose, most preferably it has a degree of polymerization of from 2 to 10, for example a degree of polymerization of from 2 to 8.

The term "sn-2 palmitate" as used herein refers to palmitic acid in the sn-2 position of the triglyceride to which it is bonded.

"High sn-2 palmitate triglyceride" refers to a triglyceride (TG) containing more than 30% of the palmitic acids in the sn-2 position. For example a commercially available high sn-2 palmitate ingredient is sold by Lipid Nutrition is Betapol^{™} B-55. It is a triglyceride mixture derived from vegetable oil in which at least 54% of the palmitic acid is in the sn-2 position of the glycerol molecule.

"Alpha-Lactalbumin" refers to a high-quality, easy-to-digest whey protein that comprises 20-25% of total human breast milk (HBM) protein and is the primary protein found in HBM . The structure of alpha-lactalbumin is comprised of 123 amino acids and 4 disulfide bridges and the protein has a molecular weight of 14.2K Daltons. Alpha-lactalbumin is ideal for lower protein infant formulas due to its high content of essential amino acids, particularly tryptophan.

The term "prebiotic" means non-digestible carbohydrates that beneficially affect the host by selectively stimulating the growth and/or the activity of healthy bacteria such as bifidobacteria in the colon of humans (Gibson GR, Roberfroid MB. Dietary modulation of the human colonic microbiota: introducing the concept of prebiotics. J Nutr. 1995;125:1401-12*).*

The term "probiotic" means microbial cell preparations or components of microbial cells with a beneficial effect on the health or well-being of the host. (Salminen S, Ouwehand A. Benno Y. et al. "Probiotics: how should they be defined" Trends Food Sci. Technol. 1999:10 107-10). The microbial cells are generally bacteria or yeasts.

The term "cfu" should be understood as colony-forming unit.

The term "non-rotavirus-associated" diarrhoea (also equally named "non-rotavirus-related diarrhoea" or "non-rotavirus-caused diarrhoea") indicates diarrhoea and/or very soft/semi-liquid/liquid stools non caused/associated/ related to the infection of the subject by a rotavirus (family reoviridae, subfamily Sedoreovirinae, genus Rotavirus). Non-rotavirus-associated diarrhoea are often associated with non-rotavirus-associated gastroenteritis and the invention may extend to non-rotavirus-associated gastroenteritis.

Exclusive breast feeding / infants or young children exclusively breast fed : has the common meaning of infants for which great majority of nutrients and/or energy originates from human breast milk (the "great majority" can be at least 90% or at least 95%, or at least 99%).

Infants/young children predominantly fed infant formula : has the common meaning and refers to infants or young children which nutritional sources of nutrients and/or energy predominantly originates from synthetic infant formula, follow-on milk or growing-up milks. Predominantly refers to at least 50% of those nutrients and/or energy, or at least 75%.

All percentages are by weight unless otherwise stated.

The invention will now be described in further details. It is noted that the various aspects, features, examples and embodiments described in the present application may be compatible and/or combined together.

In addition, in the context of the invention, the terms "comprising" or "comprises" do not exclude other possible elements. The composition of the present invention, including the many embodiments described herein, can comprise, consist of, or consist essentially of the essential elements and limitations of the invention described herein, as well as any additional or optional ingredients, components, or limitations described herein or otherwise depending on the needs.

### Detailed description of the invention

Typically, an infant formula in a ready-to-consume liquid form (for example reconstituted from a powder) provides 60-70 kcal/100 ml. Infant formula typically comprises, per 100 Kcal: about 1.8-4.5 g protein; about 3.3-6.0 g fat (lipids); about 300-1200 mg linoleic acid; about 9-14 g carbohydrates selected from the group consisting of lactose, sucrose, glucose, glucose syrup, starch, maltodextrins and maltose, and combinations thereof; and essential vitamins and minerals. Lactose may be the predominant carbohydrate in an infant formula. For example, a liquid infant formula may contain about 67 kcal/100 ml. In some embodiments, infant formula may comprise about 1.8-3.3 g protein per 100 Kcal. Infant formula may be in the form of a powder which can be reconstituted into a ready-to-feed liquid by adding an amount of water that results in for example a liquid having about 67 kcal/100 ml.

An infant formula may also comprise nucleotides selected from cytidine 5'-monophosphate (CMP), uridine 5'-monophosphate (UMP), adenosine 5'-monophosphate (AMP), guanosine 5'-monophosphate (GMP) and inosine 5'-monophosphate (IMP), and mixtures thereof. Infant formula may also comprise lutein, zeaxanthin, fructo-oligosaccharides, galacto-oligosaccharides, sialyl-lactose, and/or fucosyl-lactose. Long chain polyunsaturated fatty acids, such as docosahexaenoic acid (DHA) and arachidonic acid (AA) may be included in infant formula. Infant formula may also include free amino acids. Infant formula may also include other ingredients well-known in the art.

In one embodiment, the infant formula of this invention comprises about 5-6 g per 100 kcal of fat (triglycerides), with at least 8 wt% of this fat consisting of palmitic acid in the sn-2 position of a triglyceride. In some embodiments, about 8.0-11.5 wt%, about 8.5-11.0% or about 9.0-10.0 wt% of the fat is palmitic acid in the sn-2 position of a triglyceride.

In some embodiments, palmitic acid comprises from about 15 to about 25%, such as from about 15 to about 20%, of the total fatty acids content of the formula, by weight, and at least from about 30%, for example, from about 35 to about 43% of the total palmitic acid content is in the sn-2 position.

In some embodiments, the infant formula further comprises at least one omega 6 fatty acid and at least one omega 3 fatty acid in a ratio of about 6 to about 1. In one embodiment, at least one omega 6 fatty acid comprises from about 10 to about 15% by weight of the total fatty acids and at least one omega 3 fatty acid comprises from about 1.2% to about 3.6% of the total fatty acids. In some embodiments, the infant formula comprises at least one omega 6 fatty acid present from about 2 to about 4% of the total weight and at least one omega 3 fatty acid present from about 0.3% to about 0.6% of the total weight.

The fat in the infant formula of this invention comprises a variety of triglycerides typically found in milk and/or infant formula. The most common fatty acid residues in the triglycerides are palmitic and oleic acids. Fatty acid residues in addition to oleic and palmitic acids that are present include, but are not limited to linoleic acid, alpha linolenic acid, lauric acid, myristic acid, docosahexaenoic acid, and arachidonic acid. Recent infant clinical studies have shown that nutritional formulas containing at least one omega 6 fatty acid and at least one omega 3 fatty acid in a ratio of from about 6 to about 1 increased DHA accretion in erythrocytes and plasma. A balanced ratio of about 6:1 of omega 6 fatty acid to omega 3 fatty acid may also provide long term health benefits including protection against cardiovascular disease. Such balance will be achieved by formulating the present invention with vegetable oil fat sources that have omega 6 fatty acid content, such as, for example, soybean oil and sunflower oil, and omega 3 fatty acid content, for example, rapeseed, canola, flaxseed, chia, perlla or walnuts. A unique fat blend with 5 different oils will be used to achieve the modified fat blend

In one embodiment, the infant formula of this invention comprises from about 1.8 to about 2.2 g of total protein per 100 kcal, for example, about from 1.8 to about 2.1 g or from about 1.9 to about 2.1 g protein per 100 kcal, wherein from about 0.3 to about 0.4 g/100 kcal of protein is alpha-lactalbumin. The infant formula of this invention may be in the form of a ready-to-feed liquid, or may be a liquid concentrate or powdered formula that can be reconstituted into a ready-to-feed liquid by adding an amount of water that results in a liquid having about 67 kcal/100 ml. The infant formula of this invention includes all the ingredients that are required by law in the US or EU, including but not limited to certain vitamins, minerals, and essential amino acids. It may also include nucleotides, such as CMP, UMP, AMP, GMP and IMP, lutein, zeaxanthin, and other ingredients known in the art.

### Oligosaccharides

The nutritional composition of the invention comprises oligosaccharides as defined below. The mixture of oligosaccharides is herein referred to as the "oligosaccharides" or the "oligosaccharide mixture". Such oligosaccharide(s) can for example be polyfructose, fructooligosaccharides (FOS), long chain fructo-oligosaccharides, short-chain fructo-oligosaccharides (for example with degree of polymerisation (DP) between 2 and 8), inulin, galacto-oligosaccharides, sialylated-oligosaccharides, fucosylated oligosaccharides, N-acetylated oligosaccharides and mixture of thereof.

### Oligosaccharide mixture:

In one embodiment the oligosaccharides of the invention are present in the composition in an amount of between 0.5 and 10 g/100kcal, preferably between 1 and 5 g/100kcal, most preferably between 2 and 4 g/100kcal. In some specific embodiments, the nutritional composition may comprise the oligosaccharide mixture in an amount from 0.5 to 3.1 g/100kcal, or in an amount from 0.6 to 3.1 g/100kcal, or in an amount from 0.6 to 2.0 g/100kcal, or in an amount from 0.7 to 2.0 g/100kcal, or in an amount from 0.8 to 1.8 g/100kcal, or in an amount from 0.9 to 1.7 g/100kcal, or in an amount from 1.0 to 1.5 g/100kcal or in an amount from 1.0 to 1.6 g/100kcal.

In one embodiment the oligosaccharides are present in the composition in an amount of at least 0.5 w%, 1 wt%, at least 5wt% or at least 10 wt%. In one embodiment the oligosaccharides are present in the composition in an amount of between 0.5 w% and 10 wt%, or between 1 wt% and 5 wt%.

In one aspect of the invention, the nutritional composition comprises the oligosaccharide mixture in an amount from 1% or 2.5% to 15 wt%. Alternatively, the nutritional composition comprises the oligosaccharide mixture in an amount from 3 to 15 wt%, or in an amount from 3 to 10 wt%, or in an amount from 3.5 to 9.5 wt% or in an amount from 4 to 9 wt% or in an amount from 4.5 to 8.5 wt%, or in an amount from 5.0 to 7.5 wt% or in an amount from 5 to 8 wt%.

The mixture of oligosaccharides of the invention comprises N-acetylated oligosaccharide(s), and Galacto-oligosaccharide(s) (GOS), and Sialylated oligosaccharide(s). According to the present invention the composition comprises:
- N-acetylated oligosaccharides between 0.001 to 1 wt%, preferably between 0.003 wt% and 0.3 wt%
- Galacto-oligosaccharides between 1 and 10wt%, preferably between 3 and 6 wt%, and
- Sialylated oligosaccharides between 0.005 and 1 wt%, preferably between 0.01 and 0.4 wt%

### N-acetylated oligosaccharide:

The N-acetylated oligosaccharide is an oligosaccharide having an N-acetylated residue. Suitable N-acetylated oligosaccharides of the oligosaccharide mixture of the nutritional composition according to the present invention include GalNAcβ1,3Galβ1,4Glc and Galβ1,6GalNAcβ1,3Galβ1,4Glc, but also any mixture thereof. The N-acetylated oligosaccharides may be prepared by the action of glucosaminidase and/or galactoaminidase on N-acetyl-glucose and/or N-acetyl galactose. Equally, N-acetyl-galactosyl transferases and/or N-acetyl-glycosyl transferases may be used for this purpose. The N-acetylated oligosaccharides may also be produced by fermentation technology using respective enzymes (recombinant or natural) and/or microbial fermentation. In the latter case the microbes may either express their natural enzymes and substrates or may be engineered to produce respective substrates and enzymes. Single microbial cultures or mixed cultures may be used. N-acetylated oligosaccharide formation can be initiated by acceptor substrates starting from any degree of polymerization (DP) from DP=1 onwards. Another option is the chemical conversion of keto-hexose (fructose) either free or bound to an oligosaccharide (e.g lactulose) into N-acetylhexosamine or an N-acetylhexosamine containing oligosaccharide as described in Wrodnigg, T.M, Dtutz, A.E , Angew. Chem. Int. Ed. 1999: 38: 827-828.

The **galacto-oligosaccharide** is an oligosaccharide comprising two or more galactose molecules which has no charge and no N-acetyl residue.

The expressions "galacto-oligosaccharide" and "GOS" can be used interchangeably. They refer to an oligosaccharide comprising two or more galactose molecules which has no charge and no N-acetyl residue (i.e. they are neutral oligosaccharide). In a particular embodiment, said two or more galactose molecules are linked by a β-1,2, β-1,3, β-1,4 or β-1,6 linkage. In another embodiment, "galacto-oligosaccharide" and "GOS" also include oligosaccharides comprising one galactose molecule and one glucose molecule (i.e. disaccharides) which are linked by a β-1,2, β-1,3 or β-1,6 linkage. Suitable galacto-oligosaccharides of the oligosaccharide mixture of the nutritional composition according to the present invention include Galβ1,3Galβ1,4Glc, Galβ1,6Galβ1,4Glc, Galβ1,3Galβ1,3Galβ1,4Glc, Galβ1,6Galβ1,6Galβ1,4Glc, Galβ1,3Galβ1,6Galβ1,4Glc, Galβ1,6Galβ1,3Galβ1,4Glc, Galβ1,6Galβ1,6Galβ1,6Glc, Galβ1,3Galβ1,3Glc, Galβ1,4Galβ1,4Glc and Galβ1,4Galβ1,4Galβ1,4Glc, but also any mixture thereof. Synthesized galacto-oligosaccharides such as Galβ1,6Galβ1,4Glc, Galβ1,6Galβ1,6Galβ1,6Glc, Galβ1,3Galβ1,4Glc, Galβ1,6Galβ1,6Galβ1,4Glc, Galβ1,6Galβ1,3Galβ1,4Glc, Galβ1,3Galβ1,6Galβ1,4Glc, Galβ1,4Galβ1,4Glc and Galβ1,4Galβ1,4Galβ1,4Glc and mixture thereof are commercially available under trademarks Vivinal ^{®} and Elix'or^{®}. Other suppliers of oligosaccharides are Dextra Laboratories, Sigma-Aldrich Chemie GmbH and Kyowa Hakko Kogyo Co., Ltd. Alternatively, specific glycotransferases, such as galoctosyltransferases may be used to produce neutral oligosaccharides.

The **sialylated oligosaccharide** is an oligosaccharide having a sialic acid residue with associated charge. Suitable sialylated oligosaccharides of the oligosaccharide mixture of the nutritional composition according to the present invention include include sialyllactose, α2,3-sialyllactose (3SL), α2,6-sialyllactose (6SL), NeuAcα2-3Galβ1-4Glc, NeuAcα2-6Galβ1-4Glc, NeuAc 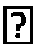 2,3Galβ1,4Glc and NeuAc 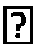 2,6Galβ1,4Glc, but also any mixture thereof.

The sialylated oligosaccharide(s) can be selected from the group comprising 3' sialyllactose (3-SL), 6' sialyllactose (6-SL), and any combination thereof. In some embodiments of the invention the composition comprises 3-SL and 6-SL. In some particular embodiments the ratio between 3'-sialyllactose (3-SL) and 6'-sialyllactose (6-SL) can be in the range between 5:1 and 1:10, or from 3:1 and 1:1, or from 1:1 to 1:10. In some specific embodiments the sialylated oligosaccharide of the composition is 6'sialyllactose (6-SL).

These sialylated oligosaccharides may be isolated by chromatographic or filtration technology from a natural source such as animal milks. Alternatively, they may also be produced by biotechnology using specific sialyltransferases either by enzyme based fermentation technology (recombinant or natural enzymes) or by microbial fermentation technology. In the latter case microbes may either express their natural enzymes and substrates or may be engineered to produce respective substrates and enzymes. Single microbial cultures or mixed cultures may be used. Sialyl-oligosaccharide formation can be initiated by acceptor substrates starting from any degree of polymerization (DP) from DP=1 onwards. Alternatively, sialyllactoses may be produced by chemical synthesis from lactose and free N'-acetylneuraminic acid (sialic acid). Sialyllactoses are also commercially available for example from Kyowa Hakko Kogyo of Japan.

### Source of prebiotics / oligosaccharides:

The oligosaccharides can be isolated from any source. Preferably the oligosaccharides are isolated, purified or concentrated from bovine milk. Alternatively all or some of the oligosaccharides are produced in totality or in part by bioengineering.

Conventional technologies for fractioning and enriching bovine milk fractions in Bovine Milk derived Oligosaccharides can be used (such conventional technologies include column filtration, resin-filtration, nano-filtration, enzymatic treatment specially with beta-galactosidase, precipitation of proteins, crystallisation and separation of lactose etc,...). Some fractions of bovine milk enriched in oligosaccharides are commercially available or have been described (for example in EP2526784 A1 which process can be used to provide the oligosaccharide mixture used by the present invention).

### Specific embodiments:

The nutritional composition of the present invention may comprise at least 0.01 wt% of N-acetylated oligosaccharide(s), at least 2.0 wt% of galacto-oligosaccharide(s) and at least 0.02 wt% of sialylated oligosaccharide(s).

In some embodiments, the nutritional composition according to the present invention may comprise at least 0.01 wt%, or at least 0.02 wt%, or at least 0.03 wt%, or at least 0.04 wt%, or at least 0.05 wt%, or at least 0.06 wt% or at least 0.07 wt% of N-acetylated oligosaccharide(s). In some embodiments, it may comprise from 0.01 to 0.07 wt% of N-acetylated oligosaccharide(s) such as from 0.01 to 0.05 wt% of N-acetylated oligosaccharide(s) or from 0.01 to 0.03 wt% of N-acetylated oligosaccharide(s).

In addition, the nutritional composition may comprise at least 2 wt%, or at least 3 wt%, or at least 4 wt%, or at least 5 wt%, or at least 5.5 wt%, or at least 6 wt% or at least 7 wt% or at least 8 wt% of galacto-oligosaccharide(s). In some embodiments, it may comprise from 5 to 8 wt% of galacto-oligosaccharide(s) such as from 5.75 to 7 wt% of galacto-oligosaccharide(s) or from 5.85 to 6.5 wt% of galacto-oligosaccharide(s). A particular example is an amount of 5.95 wt% of oligosaccharide(s).

Finally, the nutritional composition may comprise at least 0.02 wt%, or at least 0.03 wt%, or at least 0.04 wt%, or at least 0.05 wt%, or at least 0.06 wt%, or at least 0.07 wt%, or at least 0.08 wt% or at least 0.09 wt% of sialylated oligosaccharides. In some embodiments, it may comprise from 0.02 to 0.09 wt% of sialylated oligosaccharide(s) such as from 0.02 to 0.08 wt% of sialylated oligosaccharide(s), or from 0.02 to 0.07 wt% of sialylated oligosaccharide(s) or from 0.003 to 0.07 wt% of sialylated oligosaccharide(s).

In a particular embodiment, the nutritional composition according to the present invention may comprise from 0.01 to 0.07 wt% of N-acetylated oligosaccharide(s), from 2.0 to 8.0 wt% of galacto-oligosaccharide(s) and from 0.02 to 0.09 wt% of sialylated oligosaccharide(s).

In yet another particular embodiment, the nutritional composition according to the present invention may comprise from 0.01 to 0.03 wt% of N-acetylated oligosaccharide(s), 5.95 wt% galacto-oligosaccharide(s) and from 0.02 to 0.09 wt% of sialylated oligosaccharide(s).

In another embodiment, the nutritional composition may comprise at least 0.0015 g/100kcal of N-acetylated oligosaccharide(s), at least 0.70 g/100kcal of galacto-oligosaccharide(s) and at least 0.0045 g/100kcal of sialylated oligosaccharide(s).

In some specific embodiments, the nutritional composition may comprise at least 0.0015 g/100kcal, or at least 0.002 g/100kcal, or at least 0.0025 g/100kcal, or at least 0.003 g/100kcal, or at least 0.0035 g/100kcal, or at least 0.004 g/100kcal, or at least 0.0045 g/100kcal or at least 0.005 g/100kcal of N-acetylated oligosaccharide(s). In some embodiments, the nutritional composition may comprise from 0.0015 to 0.005 g/100 kcal of N-acetylated oligosaccharide(s) such as from 0.0015 to 0.045 g/100 kcal of N-acetylated oligosaccharide(s) or from 0.002 to 0.0045 g/100 kcal of N-acetylated oligosaccharide(s).

In addition the nutritional composition may comprise at least 0.70 g/100kcal, or at least 0.74 g/100kcal, or at least 0.8 g/100 kcal, or at least 0.85 g/100kcal, or at least 0.90 g/100kcal, or at least 0.95 g/100kcal, or at least 1.0 g/100kcal, or at least 1.05 g/100kcal, or at least 1.10 g/100kcal, or at least 1.20 g/100kcal or at least 1.50 of galacto-oligosaccharide(s). In some embodiments, it may comprise from 0.70 to 1.5 g/100kcal of galacto-oligosaccharide(s) such as from 0.70 to 1.20 g/100kcal of galacto-oligosaccharide(s) or from 0.74 to 1.2 g/100kcal of galacto-oligosaccharide(s).

Finally the nutritional composition may comprise at least 0.0045 g/100kcal, or at least 0.005 g/100kcal, or at least 0.0055 g/100kcal, or at least 0.006 g/100kcal, or at least 0.0065 g/100kcal, or at least 0.007 g/100kcal, or at least 0.0075 g/100kcal, or at least 0.008 g/100kcal or at least 0.0085 g/100kcal of sialylated oligosaccharide(s). In some embodiments, it may comprise from 0.0045 to 0.0085 g/100kcal of sialylated oligosaccharide(s) such as from 0.0045 to 0.008 g/100 kcal of sialylated oligosaccharide(s) or from 0.0045 to 0.0075 g/100kcal of sialylated oligosaccharide(s). In a particular embodiment, the nutritional composition may comprise from 0.0015 to 0.005 g/100kcal of N-acetylated oligosaccharide(s), from 0.70 to 1.5 g/100kcal of galacto-oligosaccharide(s) and from 0.0045 to 0.0085 g/100kcal of sialylated oligosaccharide(s).

In another particular embodiment, the nutritional composition may comprise from 0.0015 to 0.0045 g/100kcal of N-acetyl-oligosaccharide(s), from 0.74 to 1.2 g/100kcal of galacto-oligosaccharide(s) and from 0.0045 to 0.0075 g/100kcal of sialylated oligosaccharide(s).

In a particular advantageous embodiment, the oligosaccharide mixture of the nutritional composition according to the invention comprises from 0.1 to 4.0 wt% of N-acetylated oligosaccharide(s), from 92.0 to 98.5 wt% of the galacto-oligosaccharide(s) and from 0.3 to 4.0 wt% of the sialylated oligosaccharide(s).

In one aspect of the invention, the nutritional composition comprises the oligosaccharide mixture in an amount from 2.5 to 15 wt%. Alternatively, the nutritional composition comprises the oligosaccharide mixture in an amount from 3 to 15 wt%, or in an amount from 3 to 10 wt%, or in an amount from 3.5 to 9.5 wt% or in an amount from 4 to 9 wt% or in an amount from 4.5 to 8.5 wt%, or in an amount from 5.0 to 7.5 wt% or in an amount from 5 to 8 wt%.

In some specific embodiments, the nutritional composition may comprise the oligosaccharide mixture in an amount from 0.5 to 3.1 g/100kcal, or in an amount from 0.6 to 3.1 g/100kcal, or in an amount from 0.6 to 2.0 g/100kcal, or in an amount from 0.7 to 2.0 g/100kcal, or in an amount from 0.8 to 1.8 g/100kcal, or in an amount from 0.9 to 1.7 g/100kcal, or in an amount from 1.0 to 1.5 g/100kcal or in an amount from 1.0 to 1.6 g/100kcal.

The nutritional composition of the present invention may comprise at least 0.01 wt% of N-acetylated oligosaccharide(s), at least 2.0 wt% of galacto-oligosaccharide(s) and at least 0.02 wt% of sialylated oligosaccharide(s).

In some embodiments, the nutritional composition according to the present invention may comprise at least 0.01 wt%, or at least 0.02 wt%, or at least 0.03 wt%, or at least 0.04 wt%, or at least 0.05 wt%, or at least 0.06 wt% or at least 0.07 wt% of N-acetylated oligosaccharide(s). In some embodiments, it may comprise from 0.01 to 0.07 wt% of N-acetylated oligosaccharide(s) such as from 0.01 to 0.05 wt% of N-acetylated oligosaccharide(s) or from 0.01 to 0.03 wt% of N-acetylated oligosaccharide(s).

In addition, the nutritional composition may comprise at least 2 wt%, or at least 3 wt%, or at least 4 wt%, or at least 5 wt%, or at least 5.5 wt%, or at least 6 wt% or at least 7 wt% or at least 8 wt% of galacto-oligosaccharide(s).In some embodiments, it may comprise from 5 to 8 wt% of galacto-oligosaccharide(s) such as from 5.75 to 7 wt% of galacto-oligosaccharide(s) or from 5.85 to 6.5 wt% of galacto-oligosaccharide(s). A particular example is an amount of 5.95 wt% of oligosaccharide(s).

Finally, the nutritional composition may comprise at least 0.02 wt%, or at least 0.03 wt%, or at least 0.04 wt%, or at least 0.05 wt%, or at least 0.06 wt%, or at least 0.07 wt%, or at least 0.08 wt% or at least 0.09 wt% of sialylated oligosaccharides. In some embodiments, it may comprise from 0.02 to 0.09 wt% of sialylated oligosaccharide(s) such as from 0.02 to 0.08 wt% of sialylated oligosaccharide(s), or from 0.02 to 0.07 wt% of sialylated oligosaccharide(s) or from 0.003 to 0.07 wt% of sialylated oligosaccharide(s).

In a particular embodiment, the nutritional composition according to the present invention may comprise from 0.01 to 0.07 wt% of N-acetylated oligosaccharide(s), from 2.0 to 8.0 wt% of galacto-oligosaccharide(s) and from 0.02 to 0.09 wt% of sialylated oligosaccharide(s).

In yet another particular embodiment, the nutritional composition according to the present invention may comprise from 0.01 to 0.03 wt% of N-acetylated oligosaccharide(s), 5.95 wt% galacto-oligosaccharide(s) and from 0.02 to 0.09 wt% of sialylated oligosaccharide(s).

In another embodiment, the nutritional composition may comprise at least 0.0015 g/100kcal of N-acetylated oligosaccharide(s), at least 0.70 g/100kcal of galacto-oligosaccharide(s) and at least 0.0045 g/100kcal of sialylated oligosaccharide(s).

In some specific embodiments, the nutritional composition may comprise at least 0.0015 g/100kcal, or at least 0.002 g/100kcal, or at least 0.0025 g/100kcal, or at least 0.003 g/100kcal, or at least 0.0035 g/100kcal, or at least 0.004 g/100kcal, or at least 0.0045 g/100kcal or at least 0.005 g/100kcal of N-acetylated oligosaccharide(s). In some embodiments, the nutritional composition may comprise from 0.0015 to 0.005 g/100 kcal of N-acetylated oligosaccharide(s) such as from 0.0015 to 0.045 g/100 kcal of N-acetylated oligosaccharide(s) or from 0.002 to 0.0045 g/100 kcal of N-acetylated oligosaccharide(s).

In addition the nutritional composition may comprise at least 0.70 g/100kcal, or at least 0.74 g/100kcal, or at least 0.8 g/100 kcal, or at least 0.85 g/100kcal, or at least 0.90 g/100kcal, or at least 0.95 g/100kcal, or at least 1.0 g/100kcal, or at least 1.05 g/100kcal, or at least 1.10 g/100kcal, or at least 1.20 g/100kcal or at least 1.50 of galacto-oligosaccharide(s). In some embodiments, it may comprise from 0.70 to 1.5 g/100kcal of galacto-oligosaccharide(s) such as from 0.70 to 1.20 g/100kcal of galacto-oligosaccharide(s) or from 0.74 to 1.2 g/100kcal of galacto-oligosaccharide(s).

### Oligosaccharides / BMOS :

In one embodiment of the invention, the composition comprises "Bovine Milk Oligosaccharides" (herein abbreviated "BMOS" or "BMOs mixture") which is a mixture (fraction from bovine milk enriched in certain oligosaccharides) comprising :
The BMOS provides (in the final composition such as an infant formula, on a dry weight basis) approximately:

| | |
|---|---|
| N-acetylated oligosaccharides: | from 0.006 to 0.24 wt% |
| Galacto-oligosaccharides: | from 5.52 to 5.91 wt% |
| Sialylated oligosaccharides: | from 0.018 to 0.24 wt% |

The GOS can be commercial "Vivinal GOS" sourced from Friesland Campina (NL).

In various embodiments the GOS comprises less than 30, 20, 10, 8, 6, 5, or 4 units of galactose.

In a particular embodiment, the nutritional composition comprises from 2.5 to 15.0 wt% of the BMOS mixture.

In another embodiment, the nutritional composition comprises at least 0.01 wt% of N-acetylated oligosaccharide(s), at least 2.0 wt% of galacto-oligosaccharide(s) and at least 0.02 wt% of sialylated oligosaccharide(s).

In one embodiment the composition comprises at least one N-acetylated oligosaccharide, and at least one galacto-oligosaccharide, and at least one sialylated oligosaccharide.

In one embodiment, the BMOS mixture comprises from 0.1 to 4.0 wt% of the N-acetylated oligosaccharide(s), from 92.0 to 98.5 wt% of the galacto-oligosaccharide(s) and from 0.3 to 4.0 wt% of the sialylated oligosaccharide(s)._

In one embodiment the BMOS mixture is derived from animal milk, such as cow's milk, or buffalo's milk.

The nutritional composition may comprise at least 0.0045 g/100kcal, or at least 0.005 g/100kcal, or at least 0.0055 g/100kcal, or at least 0.006 g/100kcal, or at least 0.0065 g/100kcal, or at least 0.007 g/100kcal, or at least 0.0075 g/100kcal, or at least 0.008 g/100kcal or at least 0.0085 g/100kcal of sialylated oligosaccharide(s). In some embodiments, it may comprise from 0.0045 to 0.0085 g/100kcal of sialylated oligosaccharide(s) such as from 0.0045 to 0.008 g/100 kcal of sialylated oligosaccharide(s) or from 0.0045 to 0.0075 g/100kcal of sialylated oligosaccharide(s).

In a particular embodiment, the nutritional composition may comprise from 0.0015 to 0.005 g/100kcal of N-acetylated oligosaccharide(s), from 0.70 to 1.5 g/100kcal of galacto-oligosaccharide(s) and from 0.0045 to 0.0085 g/100kcal of sialylated oligosaccharide(s).

In another particular embodiment, the nutritional composition may comprise from 0.0015 to 0.0045 g/100kcal of N-acetyl-oligosaccharide(s), from 0.74 to 1.2 g/100kcal of galacto-oligosaccharide(s) and from 0.0045 to 0.0075 g/100kcal of sialylated oligosaccharide(s).

In a particular advantageous embodiment, the oligosaccharide mixture of the nutritional composition according to the invention comprises from 0.1 to 4.0 wt% of N-acetylated oligosaccharide(s), from 92.0 to 98.5 wt% of the galacto-oligosaccharide(s) and from 0.3 to 4.0 wt% of the sialylated oligosaccharide(s).

WO2006087391 and WO2012160080 provide some examples of production of BMOS mixtures.

### Other Oligosaccharides / Oligofructose (OF)

The infant formula of this invention can comprise at least about 0.4 g or at least 0.7 g of oligofructose per 100 kcal of the composition. In some embodiments, it contains from about 0.4 to about 0.9 g, from about 0.4 to about 0.7 g, from about 0.4 to about 0.5 g, from about 0.7 to about 0.8 g, or from about 0.7 to about 0.9 g, oligofructose per 100 kcal.

In some embodiments the oligofructose has a degree of polymerization of from 2 to 10. In some embodiments, at least 80%, 90%, 95%, 99% or 100% of the oligofructose has a degree of polymerization of from 2 to 8 (or between 2 and 8). In one embodiment the FOS is a long-chain fructo-oligosaccharide (chain of 10 more or 15 or more fructose units), such as Inulin.

In one embodiment the composition of the invention comprises
- at least 3 g / L or at least 5 g / L of Oligofructose (OF) when the composition is a ready-to-drink liquid composition, or
- a sufficient amount of oligosaccharide to obtain respectively at least 3 g/L or 5g/L of oligofructose in the reconstituted composition when said nutritional composition is a powered or concentrated composition.

In some embodiments the nutritional composition of the invention comprises at least 0.4 g OF /100 kcal of composition or at least 0.7 g, or at least 0.75g, or at least 0.8 g or at least 0.9 g OF /100 kcal of composition.

It is generally admitted, in view of the results illustrated by the examples, that a high amount of oligofructose delivers a stronger effect. A upper limit for a beneficial effect of oligofructose may; however, exist when disadvantageous side effect begins. Such upper limit may be for example 2.2 g / 100kcal, 2.0g / 100kcal, 1.8 g /100kcal, 1.5 g /100kcal, or 1.2 g /100kcal . Preferably the composition of the invention comprises 5 g OF / L or 0.75 or 0.9 g OF/100kcal of composition or at last such amounts.

### Other Prebiotics:

The composition of the invention can comprise said or further non-digestible oligosaccharides (e.g. prebiotics). They are usually in an amount between 0.3 and 10% by weight of composition.

Prebiotics are usually non-digestible in the sense that they are not broken down and absorbed in the stomach or small intestine and thus remain intact when they pass into the colon where they are selectively fermented by the beneficial bacteria. Examples of prebiotics include certain oligosaccharides, such further fructo-oligosaccharides (FOS) and/or galacto-oligosaccharides (GOS). A combination of prebiotics may be used such as 90% GOS with 10% short chain fructo-oligosaccharides. Another combination of prebiotics is 70% short chain fructo-oligosaccharides and 30% inulin (= long chain FOS). Both, as well as oligofructose (OF), are available commercially, in particular from the company BENEO (Beneo GmbH, Maximilianstrasse, 68165, Mannheim, Germany).

### Probiotics

The composition of the invention comprises *Bifidobacterium animalis spp. lactis* (*B. lactis*) which is a probiotic.

In one embodiment the probiotic *B. lactis* is commercial "BB12" ^{™} available from CHr. Hansen, Denmark. In one embodiment the probiotic is *Bifidobacterium animalis spp. lactis (B. lactis) probiotic* is CNCM I-3446.

The dosage of probiotics can be for example between 10⁵ and 10¹² cfu per gram of composition, preferably in an amount sufficient to deliver a synergistic effect with the oligosaccharides (e.g. BMOS) of the composition, and preferably between 10⁶ and 10⁸ cfu/g of composition.

### Proteins / Alpha-lactalbumin

The composition of the invention comprises a source of protein. Such protein source can, for example, deliver between 1.6 g and 3 g protein/100kcal. In one embodiment intended for premature infants, such amount can be between 2.4 and 4 g/100kcal or more than 3.6 g/100kcal. In one embodiment, the amount can be below 2.0 g per 100 kcal, e.g. in an amount below 1.8 g per 100 kcal.

The type of protein is not believed to be of highest criticality to the present invention provided that the minimum requirements for essential amino acid content are met and satisfactory growth is ensured. However particular proteins can provide a most suitable substrate for the microbiota. Thus, protein sources based on whey, casein and mixtures thereof may be used as well as protein sources based on soy. As far as whey proteins are concerned, the protein source may be based on acid whey or sweet whey or mixtures thereof and may include alpha-lactalbumin and beta-lactoglobulin in any desired proportions.

Preferably the protein source is whey predominant (more than 50% of proteins are coming from whey proteins). In one embodiment, the protein of the composition are intact proteins or mostly (more than 90%) intact proteins.

The proteins may be intact or hydrolysed or a mixture of intact and hydrolysed proteins. By the term "intact" is meant that the main part of the proteins are intact, i.e. the molecular structure is not altered, for example, at least 80% of the proteins are not altered, such as at least 85% of the proteins are not altered, preferably at least 90% of the proteins are not altered, even more preferably at least 95% of the proteins are not altered, such as at least 98% of the proteins are not altered. In a particular embodiment, 100% of the proteins are not altered.

The term "hydrolysed" means in the context of the present invention a protein which has been hydrolysed or broken down into its component amino acids.

The proteins may be either fully , extensively or partially hydrolysed. It may be desirable to supply partially hydrolysed proteins (degree of hydrolysis between 2 and 20%), for example, for infants believed to be at risk of developing cow's milk allergy. If hydrolysed proteins are required, the hydrolysis process may be carried out as desired and as is known in the art. For example, whey protein hydrolysates may be prepared by enzymatically hydrolysing the whey fraction in one or more steps. If the whey fraction used as the starting material is substantially lactose free, it is found that the protein suffers much less lysine blockage during the hydrolysis process. This enables the extent of lysine blockage to be reduced from about 15% by weight of total lysine to less than about 10% by weight of lysine; for example about 7% by weight of lysine which greatly improves the nutritional quality of the protein source.

In one preferable embodiment, the proteins of the composition are hydrolyzed, fully hydrolyzed, extensively hydrolyzed or partially hydrolyzed. The degree of hydrolysis (DH) of the protein can be between 8 and 40, or between 20 and 60 or between 20 and 80 or more than 10, 20, 40, 60, 80 90. It is understood that hydrolysed proteins can have several effects on allergy: hydrolyzed proteins can be less allergenic, hence triggering less immune allergic reactions. Hydrolyzed proteins, especially small peptides (of less than 20, 10 or 5 amino acids), can induce oral tolerance hence influencing the future allergic status of the subject. It is understood that hydrolyzed proteins can advantageously combine with the fucosylated oligosaccharide(s) of the present invention by providing a dual effect, possibly synergistic effect by acting at least at 2 different levels in the establishment of allergic symptoms or allergic status.

In an embodiment of the invention at least 70% of the proteins are hydrolysed, preferably at least 80% of the proteins are hydrolysed, such as at least 85% of the proteins are hydrolysed, even more preferably at least 90% of the proteins are hydrolysed, such as at least 95% of the proteins are hydrolysed, particularly at least 98% of the proteins are hydrolysed. In a particular embodiment, 100% of the proteins are hydrolysed.

In one embodiment, the hydrolyzed proteins are the sole source of protein (i.e. 100% or at least 90% of protein are hydrolyzed).

In one embodiment, the hydrolyzed proteins are the primary source of protein (i.e. at least 50%, preferably 60% of proteins are hydrolyzed).

In one embodiment, the nutritional composition of the invention comprise alpha-lactalbumin in an amount of at least 0.2 or 0.3 or 0.4 g /100kcal or at least 1.7 g , or 2.0 or 2.3, or 2.6 g / L. The presence of alpha-lactalbumin in a certain amount is believed to enhance the effect of the oligofructose by providing, for example, an adequate nutritional substrate to the microbiota.

It can be contemplated that hydrolyzed protein are easier to digest and hence combine synergistically with the establishment of a gut microbiota that is close to the microbiota of breast fed infants, especially for fragile infants.

### Preferred nutritional composition matrix:

The composition according to the invention can be a synthetic nutritional composition. It can be an infant formula, a starter infant formula, a follow-on formula, a preterm formula or a fortifier such as a human milk fortifier, or a supplement. Preferably the composition of the invention is an infant formula, or a fortifier or a supplement intended for the first 4 or 6 months of age. The composition of the invention may be a commercial conventional infant formula to which the particular mixture of the oligosaccharide of claim 1 is added.

### Fat / High sn-2 palmitate:

In one embodiment, the nutritional composition comprises triglycerides with high sn-2 palmitate, preferably triglycerides having more than 33% of the palmitic acids in sn-2 position.

In one embodiment, the nutritional composition of this invention comprises about 5-6 g per 100 kcal of fat (triglycerides), with at least 8 wt% of this fat consisting of palmitic acid in the sn-2 position of a triglyceride. According to the present invention at least 8%, preferably at least 9.6% of the fat is sn-2 palmitate.

In some embodiments, about 8.0-11.5 wt%, about 8.5-11.0% or about 9.0-10.0 wt% of the fat is palmitic acid in the sn-2 position of a triglyceride.

In some embodiments, palmitic acid comprises from about 15 to about 25%, such as from about 15 to about 20%, of the total fatty acids content of the formula, by weight, and at least from about 30%, for example, from about 35 to about 43% of the total palmitic acid content is in the sn-2 position.

In some embodiments, the nutritional composition further comprises at least one omega 6 fatty acid and at least one omega 3 fatty acid in a ratio of about 6 to about 1. In one embodiment, at least one omega 6 fatty acid comprises from about 10 to about 15% by weight of the total fatty acids and at least one omega 3 fatty acid comprises from about 1.2% to about 3.6% of the total fatty acids. In some embodiments, the infant formula comprises at least one omega 6 fatty acid present from about 2 to about 4% of the total weight and at least one omega 3 fatty acid present from about 0.3% to about 0.6% of the total weight.

The fat in the nutritional composition of this invention comprises a variety of triglycerides typically found in milkand/or nutritional composition. The most common fatty acid residues in the triglycerides are palmitic and oleic acids. Fatty acid residues in addition to oleic and palmitic acids that are present include, but are not limited to linoleic acid, alpha linolenic acid, lauric acid, myristic acid, docosahexaenoic acid, and arachidonic acid.

A commercially available composition sold by Lipid Nutrition is Betapol^{™} B-55, which is a triglyceride mixture derived from vegetable oil in which at least 54% of the palmitic acid is in the sn-2 position of the glycerol molecule. In one embodiment, the fat content of the composition of the invention is about 40-50% Betapol^{™} B-55 by weight, for example, from about 43% to about 45% by weight. Those skilled in the art will appreciate that the percentage of the high sn-2 fat used and the total amount of sn-2 palmitate in the formula may vary, and that a different high sn-2 palmitate oil may be used, without departing from the spirit and scope of the invention.

Although feeding an infant a formula containing a high percentage of sn-2 palmitate helps to produce softer stools and growth of bifidobacteria in the colon, the combination of high sn-2 palmitate with oligofructose provides significantly superior stool softening while inducing an optimal gut microbiota balance and enhanced down-regulation or reduction of pathogenic bacteria load in the colon of formula-fed infants.

### Health Effect

The composition of the invention has the positive health effect of and is for use in reducing the risk or the severity or the frequency or occurrence of non-rotavirus associated diarrhea.

In one embodiment the composition of the invention reduces the risk of non-rotavirus associated diarrhea. This may encompass the comparison between 2 groups of subjects (e.g. infants) one receiving the composition of the invention as infant formula (preferably as the sole source of nutrition/energy), the other group receiving conventional infant formula (not comprising the composition of the invention) and measuring the frequency or occurrence of non-rotavirus associated diarrhea over an extended period of time (such as 1 week, 1 months, 4 or 6 months). The subjects receiving the composition of the invention may have a lower frequency of occurrence of non-rotavirus associated diarrhea or a lower severity of such occurrence. This indicates a "protective effect" of the composition of the invention, i.e. a reduction of the risk.

In one embodiment the composition of the invention reduces the severity of non-rotavirus associated diarrhea. Such severity can be indicated by a lower duration of diarrheic episodes, by more solid stools / less liquid stools, less pain, less fever, less symptoms of gastroenteritis (always by comparing a group receiving the composition of the invention to a group non receiving the composition of the invention).

In one embodiment the composition of the invention reduces the occurence of non-rotavirus associated diarrhea. The occurrence or frequency of occurrence indicates less frequent diarrheic episodes (always by comparing a group receiving the composition of the invention to a group non receiving the composition of the invention).

In embodiments of the invention the above positive health effect of the invention is observed in infant between 0 and 6 months, 0 and 12 months, or 0 and 36 months. The duration of the feeding of the infants can be 1, 2, 3, 6, 12 months, preferably the first 1,2, 3, 6, 12 months of life of the infants.

The composition of the invention can have a positive effect on the microbiota of the subject infants or young children. Such positive effect can comprise the down regulation, decrease or inhibition of growth of pathogenic bacteria and/or the up regulation, increase or promotion of growth of beneficial bacteria.

In one embodiment the health effect of the composition of the invention comprises promoting or inducing a gut microbiota that is closer to the microbiota of infants fed exclusively with human breast milk, in comparison to the microbiota of infants fed predominantly with a conventional nutritional composition (or infant formula) not comprising said probiotic. Such effect is in infants or young children between 0 and 36 months, optionally between 0 and 12 months of age.

The health effect can be observed after a few days or weeks of use of the composition - for example after 4 weeks or 6 weeks or 8 weeks of use. The It may however take 4, 6, 8 weeks before the induced microbiota to be observed.

In that context the health effect may bring the microbiota of the infants or young children closer to the microbiota of (exclusively) breast-fed infants or young children. This is especially observed when comparing to infants or young children not receiving the composition of the invention.

The microbiota induced is specific around 2 dimensions : Quantitatively the gut flora comprises more beneficial bacteria and less non-beneficial or detrimental bacteria. Qualitatively the variety of bacterial taxa resemble more to a microbiota of breast-fed infants.

By down-regulating, decreasing and/or inhibiting the growth of populations of pathogenic bacteria, and/or inducing more beneficial bacteria, qualitatively and quantitatively, the composition of the invention may provide positive health effects. Such a healthy gut / intestinal microbiota is ultimately linked to proper nutrient absorption, adequate growth, less colic, less infection, and the best gut health.

The health effect induced by the composition of the invention can, in one embodiment, be characterized by comprising an up-regulation of the population of B. *animalis* and/or Bifidobacterium, and/or B. Longum and/or Lactobacillus, and/or a down regulation of the populations of *Coprobacillus* and/or *Streptococcus* .

The effect of the invention can be preventive (for example by reducing the risk of non-rotavirus related diarrhoea and possibly also avoiding the imbalance of the gut microbiota, avoiding gut infections, maintaining a healthy intestinal microbiota, inducing a healthy intestinal microbiota) or curative (for example by reducing the severity of non-rotavirus related diarrhoea and possibly also restoring a healthy gut microbiota when it is impaired, helping eliminate or decrease pathogenic populations in the gut / intestine, inducing a healthy microbiota after impairments due, for example, to the diarrhoea).

The health effect related to the infant can be measured by measuring the frequency or severity of diarrheic episodes and comparing to reference groups (non receiving the composition of the invention).

The health effect related to the infant can also be measured by various methods as illustrated in the example below. In one embodiment however the microbiota effect is measured by the average distribution of the UNIFRAC distances (see below).

In one embodiment the health effect "promoting or inducing a gut microbiota that is closer to the microbiota of infants fed exclusively with human breast milk" is further characterized by promoting or inducing a gut microflora that has a phylogenetic distance to the microbiota of breast fed infants of less than 0.3 units (measured by Unifrac method), preferably less than 0.25 units.

Non-rotavirus associated diarrhoea are accompanied by an increase in the population of streptococcus and E. Coli bacteria as well as a decrease in the populations of bifidobacteria (Jin et al, BMC Microbiology, 2013, 13:141 and Shields et al, Infect. Dis. Clin. Pract. 2012;20:357-358). The inventors have evidenced that the composition of the invention have a contrary effect (increase of bifidobacteria; decrease of E. Coli and Streptococcus populations). This makes surprisingly the composition of the invention as a composition of choice for reducing the risk and/or severity and/or occurrence of non-rotavirus associated diarrhoea.

### Target Infants

According to the present invention, the nutritional composition is for use in infants and young children who are preterm infants, infants born small for gestational age or by Caesarean-section, hospitalized infants or infants treated or having been treated by antibiotics.

In one embodiment the target infants are infants already suffering (have having suffered at least once, at least twice, preferably in the past 2 or 6 months) from gastroenteritis and/or non-rotavirus associated diarrhoea.

In one embodiment, the infants or young children are born at term. In one embodiment, the infants or young children are born pre-mature (preterm). In one embodiment, the infants or young children are born small for gestational age. In one embodiment, the infants or young children are vaginally delivered. In one embodiment, the infants or young children are delivered by C-section. It is foreseen that the composition of the invention is even more beneficial to infants born with possibly impaired gut microbiota or fragile infants (such as prematurely born infants and/or infants born by C-section). It is also foreseen that the composition of the invention is even more beneficial to infants exhibiting intestinal disorders (such as diarrhea, infections or colic) after birth, for example, during the first 4 weeks after birth.

In embodiments of the invention, the infants are born prematurely or small for gestational age or born by caesarean section, or exhibit unbalanced or abnormal intestinal microbiota or suffer from intestinal infection; optionally, said above conditions are targeted by the composition of the invention when the infants are 0-6 months of age. Without being bound by the theory, it is believed that younger infants benefit even more from the invention, especially when the infants have (or are at risk of having) an "unbalanced intestinal microbiota" and/or have a fragile health condition (as exemplified by the conditions cited above).

In such infants or young children, acquiring a gut microbiota that is close to the gut microbiota of breast fed infant (preferably exclusively breast fed infants) is of particular interest. Indeed it provides them with a good number of health elements that can be beneficial, especially for those fragile infants.

In one embodiment, the infants and young children are 0-6 months, or 0-12 months and according to the present invention are 0-36 months of age. It is foreseen that the composition of the invention may be even more beneficial to infants just after birth (0-4 weeks or 0-8 weeks) as their intestinal tract may be more fragile.

The following examples are presented to illustrate certain embodiments and features of the present invention, but should not be construed as limiting the scope of this invention.

### Intended Feeding regimen

In one embodiment, the composition of the invention is fed to the infant or young children (or intended to be fed or instructed to be fed) during 2, 4, 8, 12 weeks or during at least 2, 4, 8, 12 weeks. In preferable embodiments, it is fed (or intended to be fed or instructed to be fed) during the first 4, 8 or 12 weeks of the life of the infant. It is believed that starting early (at birth or close to birth) is preferred to induce the intended effect.

It is expected the health to be more prominent or to established faster when the composition of the invention is used as the exclusive source of nutrition. In one embodiment the health effect is observed as long as the composition of the invention is used to cover 50% or more, or 75% or more, of the nutritional needs (e.g. energy needs) of the target infants or young children.

### Example 1:

Table 1 provide examples of the composition of the invention.

**Table 1:**

| **Per Liter** | | **Units** | Control formula | "sn2" formula | sn2+3g/L OF formula | sn2 + 5g/L OF formula |
|---|---|---|---|---|---|---|
| Energy | | Kcal | 670 | 670 | 670 | 670 |
| Protein | | g | 13.4 | 13.4 | 13.4 | 13.4 |
| Fat | | g | 36 | 36 | 36 | 36 |
| %C16 at sn-2 | | % total fat | 2.6 | 9.6 | 9.6 | 9.6 |
| Carbohydrate | | g | 73 | 73 | 73 | 73 |
| Oligofructose | | g | 0 | 0 | 3 | 5 |
| Vitamin A (RE) | | mcg | 660 | 660 | 660 | 660 |
| Vitamin D | | mcg | 10.6 | 10.6 | 10.6 | 10.6 |
| Vitamin E (TE) | | mg | 7.4 | 7.4 | 7.4 | 7.4 |
| Vitamin K | | mcg | 67 | 67 | 67 | 67 |
| Vitamin B₁ | | mcg | 1000 | 1000 | 1000 | 1000 |
| Vitamin B₂ | | mcg | 1100 | 1100 | 1100 | 1100 |
| Vitamin B₆ | | mcg | 550 | 550 | 550 | 550 |
| Vitamin B₁₂ | | mcg | 1.8 | 1.8 | 1.8 | 1.8 |
| Niacin | | mcg | 5000 | 5000 | 5000 | 5000 |
| Folic Acid | | mcg | 107 | 107 | 107 | 107 |
| Pantothenic Acid | | mcg | 3500 | 3500 | 3500 | 3500 |
| Biotin | | mcg | 20 | 20 | 20 | 20 |
| Vitamin C | | mg | 90 | 90 | 90 | 90 |
| Choline | | mg | 100 | 100 | 100 | 100 |
| Inositol | | mg | 45 | 45 | 45 | 45 |
| Taurine | | mg | 47 | 47 | 47 | 47 |
| Lutein | | mcg | 25 | 25 | 25 | 25 |
| Carotenes | | mcg | 210 | 210 | 210 | 210 |
| Calcium | | mg | 420 | 420 | 420 | 420 |
| Phosphorous | | mg | 240 | 240 | 240 | 240 |
| Magnesium | | mg | 45 | 45 | 45 | 45 |
| Iron | | mg | 8 | 8 | 8 | 8 |
| Zinc | | mg | 6 | 6 | 6 | 6 |
| Manganese | | mcg | 50 | 50 | 50 | 50 |
| Copper | | mcg | 333 | 333 | 333 | 333 |
| Iodine | | mcg | 100 | 100 | 100 | 100 |
| Sodium | | mg | 160 | 160 | 160 | 160 |
| Potassium | | mg | 650 | 650 | 650 | 650 |
| Chloride | | mg | 433 | 433 | 433 | 433 |
| Selenium | | mcg | 14 | 14 | 14 | 14 |
| Fluonde | | mcg | 25 | 25 | 25 | 25 |
| Nucleotides | | mg | 26 | 26 | 26 | 26 |
| | CMP | mg | 13 | 13 | 13 | 13 |
| | UMP | mg | 5.0 | 5.0 | 5.0 | 5.0 |
| | AMP | mg | 4.0 | 4.0 | 4.0 | 4.0 |
| | GMP | mg | 2.0 | 2.0 | 2.0 | 2.0 |
| | IMP | mg | 2.0 | 2.0 | 2.0 | 2.0 |

The above compositions in table 1 additionally contains the oligosaccharide mixture of claim 1 (aka "BMOS" as described above), .i.e
- from 0.0015 to 0.005 g/100kcal of N-acetylated oligosaccharide(s), and
- from 0.70 to 1.5 g/100kcal of galacto-oligosaccharide(s), and
- from 0.0045 to 0.0085 g/100kcal of sialylated oligosaccharide(s).

In a further example the oligosaccharide mixtures of the example above comprises:
- from 0.1 to 4.0 wt% of N-acetylated oligosaccharide(s), and
- from 92.0 to 98.5 wt% of the galacto-oligosaccharide(s) and
- from 0.3 to 4.0 wt% of the sialylated oligosaccharide(s).

In further examples, the compositions exemplified above also comprises respectively the probiotic *Bifidobacterium animalis* spp. *lactis* (BB12 ^{™} or alternatively CNCM I-3446) in an amount of 10⁶ (control formula), 10⁸(sn2 formula), 10⁹, (sn2 formula + 3 g OF) 10¹⁰ (Sn2 formula 5g OF) cfu/g of composition.

Further examples of the composition of an nutritional composition for use according to the present invention is given below in Table 2. These compositions are given by way of illustration only. The protein source is a mixture of 60% MSWP28 and 40% casein.

**TABLE 2**

| **Nutrient** | **per 100kcal** | **per litre** |
|---|---|---|
| Energy (kcal) | 100 | 670 |
| Protein (g) | 1.83 | 12.3 |
| Fat (g) | 5.3 | 35.7 |
| Linoleic acid (g) | 0.79 | 5.3 |
| α-Linolenic acid (mg) | 101 | 675 |
| Lactose (g) | 11.2 | 74.7 |
| Prebiotic oligosaccharides | | |
| BMOS (g) (as described above) | 1.1 | 7.5 |
| Optionally : GOS (g) | 0.64 | 4.3 |
| Minerals (g) | 0.37 | 2.5 |
| Na (mg) | 23 | 150 |
| K (mg) | 89 | 590 |
| Cl (mg) | 64 | 430 |
| Ca (mg) | 62 | 410 |
| P (mg) | 31 | 210 |
| Mg (mg) | 7 | 50 |
| Mn (µg) | 8 | 50 |
| Se (µg) | 2 | 13 |
| Vitamin A (µg RE) | 105 | 700 |
| Vitamin D (µg) | 1.5 | 10 |
| Vitamin E (mg TE) | 0.8 | 5.4 |
| Vitamin K1 (µg) | 8 | 54 |
| Vitamin C (mg) | 10 | 67 |
| Vitamin B1 (mg) | 0.07 | 0.47 |
| Vitamin B2 (mg) | 0.15 | 1.0 |
| Niacin (mg) | 1 | 6.7 |
| Vitamin B6 (mg) | 0.075 | 0.50 |
| Folic acid (µg) | 9 | 60 |
| Pantothenic acid (mg) | 0.45 | 3 |
| Vitamin B12 (µg) | 0.3 | 2 |
| Biotin (µg) | 2.2 | 15 |
| Choline (mg) | 10 | 67 |
| Fe (mg) | 1.2 | 8 |
| I (µg) | 15 | 100 |
| Cu (mg) | 0.06 | 0.4 |
| Zn (mg) | 0.75 | 5 |
| *Probiotic (optional) :* | 1×10⁷ cfu/g of powder | |
| *Bifidobacterium lactis* NCC2818 (CNCM I-3446), | | |

### Example 2: Clinical study

***Nutritional intervention trial.*** At a mean age of 5 days, 115 healthy full term infants were enrolled into a nutritional intervention trial. Infants from mothers who decided not to breastfeed were randomized to either a starter infant formula (control formula **C**, n=37, 1.8 g protein / 100 kcal; whey/casein ratio 70:30) or the same formula supplemented with a prebiotic (BMOS) at a total oligosaccharide concentration of 5.7 ± 1.0 g/100 g of powder formula (8 g/L in the reconstituted formula) and a probiotic (*B. lactis* strain CNCM-I-3446 with 1×10⁷ cfu/ g of powder formula; 1.4×10⁷ cfu/L reconstituted formula) (test formula **T**, n=39) for a 12-week feeding period. Infants from mothers who decided to exclusively breastfeed were enrolled in the breastfed group (group **B**, n=39), which served as physiological reference group. The "BMOS" used in the trial is as defined in the present invention.

***Stool characteristics.*** In the per protocol analysis, the number of stools decreased from 4.9 to 2.4 stools / day over the observation period, with no difference between the groups (p>0.4).

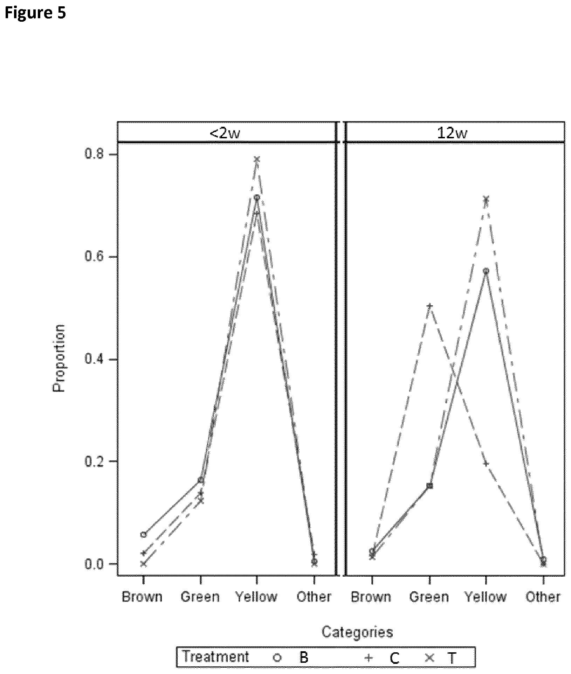

During the feeding trial, infants from the T, but not the C group showed a proportion of yellowish versus greenish stools equivalent to B infants (**Figure 5**). The color resemblance is an indicator of having similar / close microbiota.

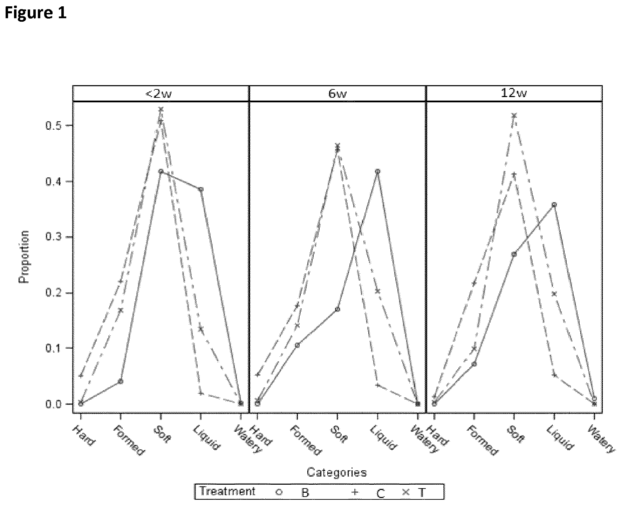

Consistency of stools: Figure 1 reports the consistency of the stools observed for the different groups of subjects (B : breast fed, C: control and T: Test group) at < 2weeks, 6 weeks and 12 weeks. The results are shown for the total test populations and show that the composition of the invention ("T" group) induces a pattern of stools consistency that is closer to the group "B" (Breastfed) than to the control group "C". At 6 weeks, infants in the T and B groups showed a similar stool pH while C infants showed a significantly higher stool pH (data not shown). Infants from the T and C groups did not differ in "spitting up", vomiting, crying, colics, flatulence, and irritability (data not shown). By being closer to the Breast fed group (B), the Test group (T) than the control group, it is interpreted that the infants fed with the composition of the invention are less susceptible to non-rotavirus related diarrhea (those infected by a rotavirus would in any case exhibit highly liquid stools).

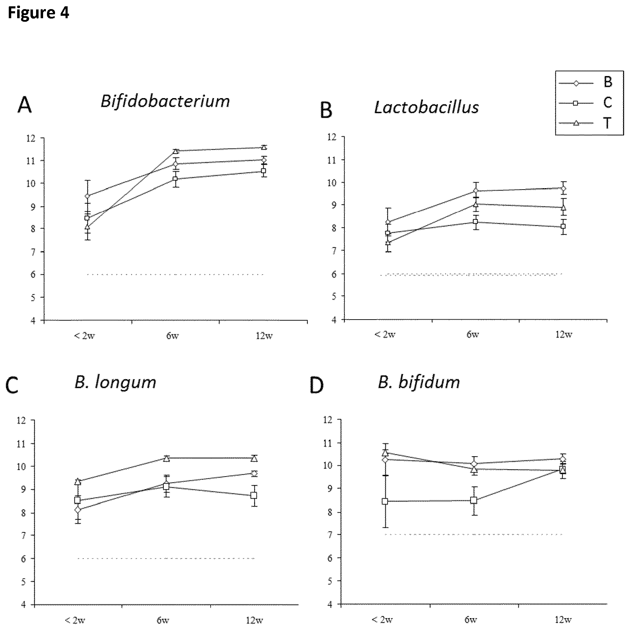

***Stool microbiology analysis: Quantitative real-time PCR.*** Stool samples were collected from infants before first product application and at 6 and 12 weeks of age when on the allotted feeding regime. In the per protocol analysis, stools from 18 to 23 infants were available per group and time point. Stool samples were collected for each time point, refrigerated at 4°C for a maximum of 10 hours after defecation and kept frozen at -80°C until the microbiota analysis was carried out. Total DNA was extracted using the QlAamp DNA Stool Mini Kit (QIAGEN), following the manufacturer's instructions, except for the addition of a series of mechanical disruption steps (11 × 45 s) using a FastPrep apparatus and Lysing Matrix B tubes (MP Biochemicals). We used 16S rRNA gene primers allowing taxonomic differentiation. Total counts of bifidobacteria increased in all three groups between enrolment and 6 weeks of age, after which it remained constant until 12 weeks (**Figure 4A**). Compared to infants in the C group, infants in the T group showed a more pronounced increase in the *Bifidobacterium* titer by 0.8 log. In the T group, the final fecal titer of bifidobacteria was with 0.8×10¹⁰ bacteria/g stool significantly higher than in the C and in the B group. *Bacteroides* and lactobacilli showed a 10-fold higher and 10-fold lower stool number, respectively, in infants from the C group compared to the T group (data not shown and **Figure 4B****).**

To achieve further resolution in the *Bifidobacterium* genus, we used a qPCR set of primers based on the *groEL* gene that allowed the detection of 12 *Bifidobacterium* species *B. bifidum, and B. longum* **(****Figures 4C and 4D****).**

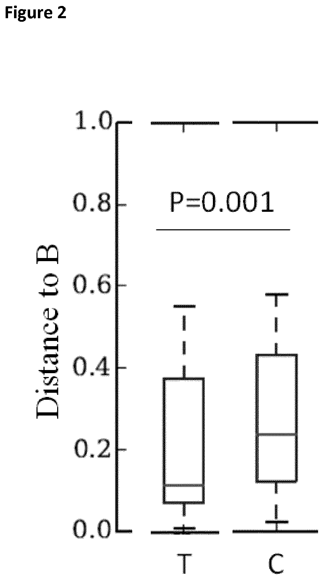

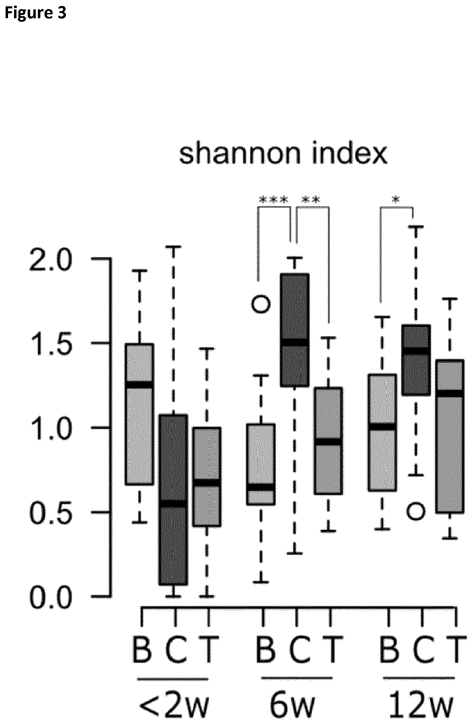

***Microbiota composition: Diversity index.*** The microbiota analysis was complemented by16S rRNA gene pyrosequencing. The 16S variable region V1 to V3 was PCR amplified and sequenced on Roche 454 GS-FLX-Titanium Sequencer. Raw sequence data were analyzed using Mothur v.1.33.0 (Schloss, P. D., S. L. Westcott, T. Ryabin, J. R. Hall, M. Hartmann, E. B. Hollister, R. A. Lesniewski, B. B. Oakley, D. H. Parks, C. J. Robinson, J. W. Sahl, B. Stres, G. G. Thallinger, D. J. Van Horn and C. F. Weber (2009). "Introducing mothur: Open-source, platform-independent, community-supported software for describing and comparing microbial communities." Applied and Environmental Microbiology 75(23): 7537-7541.) and QIIME v.1.8 (Caporaso,J.G., Kuczynski,J., Stombaugh,J., Bittinger,K., Bushman,F.D., Costello,E.K. et al. (2010b) QIIME allows analysis of high-throughput community sequencing data. Nat Methods 7: 335-336) software packages. OTUs de novo picking at 97% identity was performed and taxonomy assignment of OTU representative sequences used the RDP Classifier on the Greengenes reference database v.13.8. The same sequences were aligned using PyNast on the Greengenes core reference alignment. The resulting multiple alignments was then filtered and used to build a phylogenetic tree with FastTree. After quality filtering, phylogenetic distances between all samples were computed as UniFrac distances. After analysis and exclusion of subjects with incomplete datasets, we obtained fecal microbiota compositions for all three time points from at least 13 infants per group. **Figure 3** compares the α-diversity for the three groups at baseline and after 6 and 12 weeks of feeding (calculated with Calypso at http://bioinfo.qimr.edu.au/calypso) . At baseline the three feeding groups did not differ significantly in microbial diversity (Shannon index). At 6 weeks of age, infants in the C group showed a higher diversity index, due to an increase in both richness and evenness when compared to infants in the T group and the B group. At 12 weeks of age, the C group but not the T group differed from the B group with respect to diversity and richness. Weighted Unifrac distance (which takes into account the phylogenetic distance between OTUs) was calculated between samples using QIIME. Then, within each group, the distribution of the distances of each sample compared to the samples of the breast fed group was analysed. Statistical significance is given by Mann-Whitney U test **(****Figure 2****).** The global microbiota composition of infants fed the formula with B. *lactis* and BMOS (T) was different from the group fed the control formula (C) and phylogenetically more closely related to the group fed at breast (B).

Altogether this example demonstrates that the particular mixture of oligosaccharides as used in the invention promotes the growth in vivo of Bifidobacterium and can also decrease the growth of streptococcus and/or E.Coli, while inducing a stool pattern close to breast fed infants.

Non-rotavirus associated diarrhoea are accompanied by an increase in the population of streptococcus and E. Coli bacteria as well as a decrease in the populations of bifidobacteria (Jin et al, BMC Microbiology, 2013, 13:141 and Shields et al, Infect. Dis. Clin. Pract. 2012;20:357-358). The inventors have evidenced that the composition of the invention have a contrary effect (increase of bifidobacteria; decrease of E. Coli and Streptococcus populations). This makes surprisingly the composition of the invention as a composition of choice for reducing the risk and/or severity and/or occurrence of non-rotavirus associated diarrhoea.

### Example 4: Further clinical study (Not according to the present invention)

### Nutritional intervention; randomized double blind controlled trial.

A multi-center, randomized, controlled, double-blind trial conducted in South Africa tested the effect of a formula supplemented with a prebiotic, a mixture of bovine milk-derived oligosaccharides (BMOS) generated from whey permeate (containing galacto-oligosaccharides and milk oligosaccharides such as 3'- and 6'-sialyllactose), and the probiotic *Bifidobacterium animalis* subsp. *lactis* (*B. lactis*) strain CNCM I-3446 on *Bifidobacteria* levels in the gut of infants born vaginally or via caesarean section in early life. Additionally safety of the new formulation was evaluated. The "BMOS" used in the trial is as defined in the present invention.

A total of 430 healthy, full-term, infants born to HIV-positive mothers who had elected to feed their child exclusively with formula beginning from birth (≤3 days old) were randomized into this multicenter trial of 4 parallel groups. A total of 421 infants who had any study formula intake were included in the full analysis set (FAS).

The first two groups consisted of caesarean-delivered infants assigned to the Test formula (n= 92) (a starter IF with BMOS at total OS of 5.8 ± 1.0 g/100 g of powder formula (8 g/L in the reconstituted formula) + *B. lactis* (1 × 10⁷ cfu/g)) or a Control IF (n=101), the second two groups consisted of vaginally-delivered infants randomized to the same test (n=115) or control (n=113) formulas from enrolment to 6 months.

The primary efficacy outcome was faecal bifidobacteria counts at 10 days and the primary safety outcome was daily weight gain (g/day) between 10 days and 4 months. At 10 days, faecal bifidobacteria counts were significantly higher in the Test than in the Control formula group among infants with caesarean birth (median [range] log: 9.41 [6.30 - 10.94] CFU/g vs. 6.30 [6.30 - 10.51] CFU/g, Wilcoxon test, p = 0.002) but not among those with vaginal birth (median [range] log: 10.06 [5.93 -10.77] CFU/g vs. 9.85 [6.15 - 10.79] CFU/g, p = 0.126). The lower bound of the 2-sided 95% confidence interval of the difference in the mean daily weight gain between the Test and Control formula groups was above -3 g/day in both the vaginally and caesarean delivered infants, indicating that growth in Test formula-fed infants was not inferior to that of control formula-fed infants.

At 10 days and 4 weeks, the fecal pH of infants fed the Test formula was significantly lower than in those fed the Control formula, irrespective of mode of delivery: for vaginal delivery: 4.93 vs 5.59 p<0.001(10 days) and 5.01 vs 5.71 p< 0.001 (4 weeks), for caesarean delivery 5.14 vs 5.65 p=0.009 (10 days), 5.06 vs 5.75 p<0.001 (4 weeks) At 3 months, this acidification effect only persisted among caesarean-born infants. Infant formula supplemented with the prebiotic BMOS and probiotic *B. lactis* induces a strong bifidogenic effect in both delivering modes, but more explicitly correcting from birth the low Bifidobacterium level found in caesarean born infants. The supplemented IF lowered fecal pH and improved the fecal microbiota in both normal and caesarean-delivered infants.

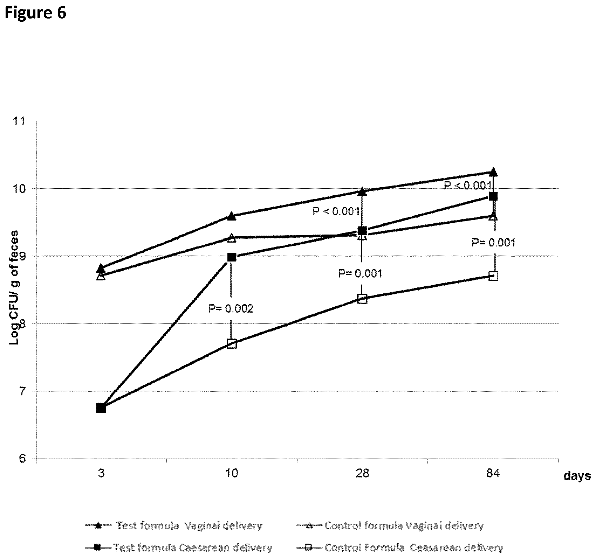

### Detailled Results: (see also figure 6)

In the FAS population, Bifidobacterium counts at 28 days and 3 months (84 days) were also significant higher in the Test formula group compared to Control group in caesarean born infants (squares on Figure 6). Then, at 28 days, the median [range] log bifidobacteria counts were 10.15 [6.30-10.96] cfu/g and 9.00 [6.30-10.77] cfu/g in the Test and Control Formula groups, respectively (non-parametric Wilcoxon test, p=0.001). At 84 days, the median [range] log bifidobacteria counts were 10.40 [6.50-10.79] cfu/g and 9.67 [6.30-10.50] cfu/g in the Test and Control Formula groups, respectively (non-parametric Wilcoxon test, p<0.001).

Among the vaginal born infants (triangles on Figure 6), the Test formula group also had increased Bifidobacterium counts significantly at 28 days and 84 days compared to the control formula group. At 28 days, median [range] log bifidobacteria counts were 10.25 [6.75-10.98] cfu/g and 9.66 [6.30-10.31] cfu/g in the Test and Control Formula groups, respectively (non-parametric Wilcoxon test, p<0.001). At 84 days, the median [range] log bifidobacteria counts were 10.45 [8.22-10.96] cfu/g and 9.95 [6.30-10.17] cfu/g in the Test and Control Formula groups, respectively (non-parametric Wilcoxon test, p<0.001).

Total bacterial counts at 3 and 10 days were not significantly different between the Test and Control formula groups among infants with either delivery mode (data not shown). However at 4 weeks, total bacterial counts were higher in the Test formula groups in both delivery groups, and at 3 months they were higher in the Test formula group only among vaginally delivered infants (data not shown).

A higher proportion of infants in the Test formula groups had detectable bifidobacteria species compared with Control formula groups up to 4 weeks (data not shown). Lactobacillus species were detectable in a higher proportion of infants at 4 weeks and 3 months in respectively caesarean and vaginally delivered infants in the Test formula groups compared to control. At 10 days, 4 weeks, and 3 months, *B. lactis* was detected in a significantly higher proportion of infants in the Test formula groups compared with those in the Control formula groups (data not shown). *E. coli* and *Staphylococcus, Enterobacteria,* and *Klebsiella* species were detected in significantly higher proportion of infants in the Control formula group compared with the Test formula group (data not shown).

## Claims

1. A nutritional composition for infants or young children, said composition comprising at least one N-acetylated oligosaccharide, and at least one galacto-oligosaccharide, and at least one sialylated oligosaccharide, in the following amount:
- N-acetylated oligosaccharides between 0.001 to 1 wt%, preferably between 0.003 wt% and 0.3 wt%
- Galacto-oligosaccharides between 1 and 10wt%, preferably between 3 and 6 wt%
- Sialylated oligosaccharides between 0.005 and 1 wt%, preferably between 0.01 and 0.4 wt%
all calculated as wt% of the composition,
for use in preventing and/or treating and/or reducing the severity and/or risk and/or occurrence of non-rotavirus-associated diarrhoea in infants or young children between 0 and 36 months, optionally between 0 and 12 months of age or between 0 and 6 months of age,
**characterized in that** said infants are preterm infants, infants born small for gestational age, infants born by Caesarean-section, hospitalized infants or infants treated or having been treated by antibiotics,
**characterized in that** said composition comprises fat and at least 8%, preferably at least 9.6% of said fat is sn2 palmitate.

2. The nutritional composition for the use of claim 1 further comprising a *Bifidobacterium animalis* spp. *lactis probiotic*, optionally CNCM-I-3446, optionally in an amount of between 10⁵ and 10¹² cfu per gram of composition, preferably between 10⁶ and 10⁸ cfu/g of composition.

3. The nutritional composition for the use according to claim 2 for use in promoting or inducing a gut microbiota that is closer to the microbiota of infants fed exclusively with human breast milk, in comparison to the microbiota of infants fed predominantly with a conventional nutritional composition not comprising said probiotic, in infants or young children between 0 and 36 months, optionally between 0 and 12 months of age.

4. The composition for the use of any of the preceding claims **characterized in that** the infants are infants suffering from gastroenteritis and/or non-rotavirus associated diarrhea.

5. The composition for the use of any of the preceding claims **characterized in that** said composition comprises oligofructose, preferably said at last 90%, 99% or 100% of said oligofructose having a degree of polymerisation between 2 and 8.

6. The composition for the use of any of the preceding claims **characterized in that** said composition comprises triglycerides having more than 33% of the palmitic acid in sn2 position.

7. The composition for the use of any of the preceding claims **characterized in that** said composition is an infant formula or a follow-on formula.

8. The composition for the use of any of the preceding claims **characterized in that** said composition comprises alpha-lactalbumin proteins in an amount of at least 0.3 g/100 kcal or 2.3 g / L of composition.

9. The composition for the use of claim 3 **characterized in that** said promoting or inducing a gut microbiota that is closer to infants fed exclusively with human breast milk is further **characterized by** comprising an up-regulation of the population of B. *animalis* and/or *Bifidobacterium,* and/or B. *Longum* and/or *Lactobacillus*, and/or a down regulation of the populations of *Coprobacillus* and/or *Streptococcus.*

10. The composition for the use of claim 3 or 9 **characterized in that** said "promoting or inducing a gut microbiota that is closer to the microbiota of infants fed exclusively with human breast milk" is further **characterized by** promoting or inducing a gut microflora that has a phylogenetic distance to the microbiota of breast fed infants of less than 0.3 units (measured by Unifrac method), preferably less than 0.25 units.

11. The composition for the use of claim 3, 9 or 10 **characterized in that** said promoting and/or inducing a gut microbiota that is closer to infants fed exclusively with human breast milk is measurable in infants stools, optionally measurable at or after 1, 4, 6 or 8 weeks of age, and optionally after 1, 4, 6 or 8 weeks of feeding with said nutritional composition.

12. The composition for the use of any of the preceding claims **characterized in that** said composition is fed or intended to be fed during the first 1, 2, 4, 8, or 12 weeks of life.

13. The composition for the use of any of claims 3, 9, 10, or 11 **characterized in that** said promoting and/or inducing a gut microbiota that is closer to infants fed exclusively with human breast milk is further **characterized by** promoting and/or inducing a healthy growth, a healthy immune system and/or a healthy gut function, especially later in life.

## Patentansprüche

1. Nährstoffzusammensetzung für Säuglinge oder Kleinkinder, wobei die Zusammensetzung mindestens ein N-acetyliertes Oligosaccharid und mindestens ein Galactooligosaccharid und mindestens ein sialyliertes Oligosaccharid umfasst, in der folgenden Menge:
- N-acetylierte Oligosaccharide 0,001 bis 1 Gew.-%, vorzugsweise 0,003 Gew.-% bis 0,3 Gew.-%
- Galactooligosaccharide 1 bis 10 Gew.-%, vorzugsweise 3 bis 6 Gew.-%
- sialylierte Oligosaccharide 0,005 bis 1 Gew.-%, vorzugsweise 0,01 bis 0,4 Gew.-%
jeweils berechnet in Gew.-% der Zusammensetzung,
zur Verwendung bei der Prävention und/oder Behandlung und/oder Verminderung des Schweregrades und/oder Risikos und/oder Auftretens von nicht mit Rotavirus assoziiertem Durchfall bei Säuglingen oder Kleinkindern im Alter von 0 bis 36 Monaten, gegebenenfalls im Alter von 0 bis 12 Monaten oder im Alter von 0 bis 6 Monaten,
**dadurch gekennzeichnet, dass** es sich bei den Säuglingen um Frühgeborene, für das Gestationsalter zu kleine Säuglinge, per Kaiserschnitt geborene Säuglinge, hospitalisierte Säuglinge oder mit Antibiotika behandelte Säuglinge handelt,
**dadurch gekennzeichnet, dass** die Zusammensetzung Fett umfasst und mindestens 8 %, vorzugsweise mindestens 9,6 % des Fetts sn2-Palmitat ist.

2. Nährstoffzusammensetzung zur Verwendung nach Anspruch 1, weiterhin umfassen ein *Bifidobacterium animalis* spp. lactis-Probiotikum, gegebenenfalls CNCM-I-3446, gegebenenfalls in einer Menge von 10⁵ bis 10¹² cfu pro Gramm der Zusammensetzung, vorzugsweise 10⁶ bis 10⁸ cfu/g der Zusammensetzung.

3. Nährstoffzusammensetzung zur Verwendung nach Anspruch 2 zur Verwendung bei der Förderung oder Induzierung einer Darmflora, die, im Vergleich zur Darmflora von überwiegend mit einer herkömmlichen, das Probiotikum nicht enthaltenden Nährstoffzusammensetzung gefütterten Säuglingen, der Darmflora von ausschließlich mit menschlicher Muttermilch gefütterten Säuglingen näher kommt, bei Säuglingen oder Kleinkindern im Alter von 0 bis 36 Monaten, gegebenenfalls im Alter von 0 bis 12 Monaten.

4. Zusammensetzung zur Verwendung nach einem der vorhergehenden Ansprüche, **dadurch gekennzeichnet, dass** es sich bei den Säuglingen um an Gastroenteritis und/oder nicht mit Rotavirus assoziiertem Durchfall leidende Säuglinge handelt.

5. Zusammensetzung zur Verwendung nach einem der vorhergehenden Ansprüche, **dadurch gekennzeichnet, dass** die Zusammensetzung Oligofructose umfasst, wobei vorzugsweise mindestens 90 %, 99 % oder 100 % der Oligofructose einen Polymerisationsgrad von 2 bis 8 hat.

6. Zusammensetzung zur Verwendung nach einem der vorhergehenden Ansprüche, **dadurch gekennzeichnet, dass** die Zusammensetzung Triglyceride mit mehr als 33 % der Palmitinsäure in der sn2-Position umfasst.

7. Zusammensetzung zur Verwendung nach einem der vorhergehenden Ansprüche, **dadurch gekennzeichnet, dass** es sich bei der Zusammensetzung um eine Säuglingsnahrung oder eine Folgenahrung handelt.

8. Zusammensetzung zur Verwendung nach einem der vorhergehenden Ansprüche, **dadurch gekennzeichnet, dass** die Zusammensetzung alpha-Lactalbumin-Proteine in einer Menege von mindestens 0,3 g/100 kcal oder 2,3 g/l der Zusammensetzung umfasst.

9. Zusammensetzung zur Verwendung nach Anspruch 3, **dadurch gekennzeichnet, dass** die Förderung oder Induzierung einer Darmflora, die der Darmflora von ausschließlich mit menschlicher Muttermilch gefütterten Säuglingen näher kommt, weiterhin **dadurch gekennzeichnet ist, dass** sie eine Hochregulierung der Population von B. *animalis* und/oder *Bifidobacterium* und/oder *B. longum* und/oder *Lactobacillus* und/oder eine Herunterregulierung der Populationen von *Coprobacillus* und/oder *Streptococcus* umfasst.

10. Zusammensetzung zur Verwendung nach Anspruch 3 oder 9, **dadurch gekennzeichnet, dass** die "Förderung oder Induzierung einer Darmflora, die der Darmflora von ausschließlich mit menschlicher Muttermilch gefütterten Säuglingen näher kommt", weiterhin durch die Förderung oder Induzierung einer Darmflora mit einer phylogenetischen Distanz zur Darmflora von gestillten Säuglingen von weniger als 0,3 Einheiten (gemessen mit der Unifrac-Methode), vorzugsweise weniger als 0,25 Einheiten, gekennzeichnet ist.

11. Zusammensetzung zur Verwendung nach Anspruch 3, 9 oder 10, **dadurch gekennzeichnet, dass** die Förderung und/oder Induzierung einer Darmflora, die der Darmflora von ausschließlich mit menschlicher Muttermilch gefütterten Säuglingen näher kommt, im Stuhl von Säuglingen messbar ist, gegebenenfalls im Alter von bzw. nach 1, 4, 6 oder 8 Wochen messbar ist und gegebenenfalls nach 1, 4, 6 oder 8 Wochen der Fütterung mit der Nährstoffzusammensetzung.

12. Zusammensetzung zur Verwendung nach einem der vorhergehenden Ansprüche, **dadurch gekennzeichnet, dass** die Zusammensetzung während der ersten 1, 2, 4, 8 oder 12 Lebenswochen gefüttert wird oder gefüttert werden soll.

13. Zusammensetzung zur Verwendung nach einem der Ansprüche 3, 9, 10 oder 11, **dadurch gekennzeichnet, dass** die Förderung und/oder Induzierung einer Darmflora, die der Darmflora von ausschließlich mit menschlicher Muttermilch gefütterten Säuglingen näher kommt, weiterhin **dadurch gekennzeichnet ist, dass** ein gesundes Wachstum, ein gesundes Immunsystem und/oder eine gesunde Darmfunktion, insbesondere im späteren Leben, gefördert bzw. induziert wird.

## Revendications

1. Composition nutritionnelle pour des nourrissons ou des jeunes enfants, ladite composition comprenant au moins un oligosaccharide N-acétylé, et au moins un galacto-oligosaccharide, et au moins un oligosaccharide sialylé, en la quantité suivante :
- oligosaccharides N-acétylés entre 0,001 et 1 % en poids, préférablement entre 0,003 % en poids et 0,3 % en poids
- galacto-oligosaccharides entre 1 et 10 % en poids, préférablement entre 3 et 6 % en poids
- oligosaccharides sialylés entre 0,005 et 1 % en poids, préférablement entre 0,01 et 0,4 % en poids tous calculés comme % en poids de la composition,
pour une utilisation dans la prévention et/ou le traitement et/ou la réduction de la gravité et/ou du risque et/ou de l'occurrence d'une diarrhée non associée à un rotavirus chez des nourrissons ou des jeunes enfants entre 0 et 36 mois, éventuellement d'un âge entre 0 et 12 mois ou d'un âge entre 0 et 6 mois,
**caractérisée en ce que** lesdits nourrissons sont des prématurés, des nourrissons nés petits pour l'âge gestationnel, des nourrissons nés par césarienne, des nourrissons hospitalisés ou des nourrissons traités ou ayant été traités par des antibiotiques,
**caractérisée en ce que** ladite composition comprend une graisse et au moins 8 %, préférablement au moins 9,6 % de ladite graisse est le palmitate sn2.

2. Composition nutritionnelle pour l'utilisation selon la revendication 1 comprenant en outre un *probiotique Bifidobacterium animalis* spp. *lactis,* éventuellement CNCM-I-3446, éventuellement en une quantité comprise entre 10⁵ et 10¹² ufc par gramme de composition, préférablement entre 10⁶ et 10⁸ ufc/g de composition.

3. Composition nutritionnelle pour l'utilisation selon la revendication 2 pour une utilisation dans la favorisation ou l'induction d'un microbiote intestinal qui est plus proche du microbiote de nourrissons alimentés exclusivement avec du lait maternel humain, par comparaison avec le microbiote de nourrissons alimentés principalement avec une composition nutritionnelle conventionnelle ne comprenant pas ledit probiotique, chez des nourrissons ou des jeunes enfants entre 0 et 36 mois, éventuellement d'un âge entre 0 et 12 mois.

4. Composition pour l'utilisation selon l'une quelconque des revendications précédentes **caractérisée en ce que** les nourrissons sont des nourrissons souffrants d'une gastro-entérite et/ou de diarrhée non associée à un rotavirus.

5. Composition pour l'utilisation selon l'une quelconque des revendications précédentes **caractérisée en ce que** ladite composition comprend un oligofructose, préférablement lesdits au moins 90 %, 99 % ou 100 % dudit oligofructose ayant un degré de polymérisation entre 2 et 8.

6. Composition pour l'utilisation selon l'une quelconque des revendications précédentes **caractérisée en ce que** ladite composition comprend des triglycérides ayant plus de 33 % de l'acide palmitique dans une position sn2.

7. Composition pour l'utilisation selon l'une quelconque des revendications précédentes **caractérisée en ce que** ladite composition est une préparation pour nourrissons ou une préparation de suite.

8. Composition pour l'utilisation selon l'une quelconque des revendications précédentes **caractérisée en ce que** ladite composition comprend des protéines d'alpha-lactalbumine en une quantité d'au moins 0,3 g/100 kcal ou 2,3 g/L de composition.

9. Composition pour l'utilisation selon la revendication 3 **caractérisée en ce que** ladite favorisation ou induction d'un microbiote intestinal qui est plus proche de nourrissons alimentés exclusivement avec du lait maternel humain est en outre **caractérisée par le fait qu'**elle comprend une régulation à la hausse de la population de *B. animalis* et/ou de *Bifidobacterium,* et/ou de *B. Longum* et/ou de *Lactobacillus* et/ou une régulation à la baisse des populations de *Coprobacillus* et/ou de *Streptococcus.*

10. Composition pour l'utilisation selon la revendication 3 ou 9 **caractérisée en ce que** ladite « favorisation ou induction d'un microbiote intestinal qui est plus proche du microbiote de nourrissons alimentés exclusivement avec du lait maternel humain » est en outre **caractérisée par** la favorisation ou l'induction d'une microflore intestinale qui possède une distance phylogénétique par rapport au microbiote de nourrissons alimentés au sein de moins de 0,3 unité (mesurée par le procédé Unifrac), préférablement de moins de 0,25 unité.

11. Composition pour l'utilisation selon la revendication 3, 9 ou 10 **caractérisée en ce que** ladite/lesdites favorisation et/ou induction d'un microbiote intestinal qui est plus proche de nourrissons alimentés exclusivement avec du lait maternel humain est/sont mesurable(s) dans des selles de nourrissons, éventuellement mesurable(s) à ou après un âge de 1, 4, 6 ou 8 semaines, et éventuellement après 1, 4, 6 ou 8 semaines d'alimentation avec ladite composition nutritionnelle.

12. Composition pour l'utilisation selon l'une quelconque des revendications précédentes **caractérisée en ce que** ladite composition est alimentée ou est prévue d'être alimentée pendant les premières 1, 2, 4, 8 ou 12 semaines de vie.

13. Composition pour l'utilisation selon l'une quelconque des revendications 3, 9, 10 ou 11 **caractérisée en ce que** ladite/lesdites favorisation et/ou induction d'un microbiote intestinal qui est plus proche de nourrissons alimentés exclusivement avec du lait maternel humain est/sont en outre **caractérisée**(s) par la favorisation et/ou l'induction d'une croissance saine, d'un système immunitaire sain et/ou d'une fonction intestinale saine, notamment plus tard dans la vie.
